# EUROPEAN PATENT SPECIFICATION

(11) **EP 1 242 821 B1**
(45) Date of publication and mention of the grant of the patent: **20.02.2008**
(21) Application number: 00977784.8
(22) Date of filing: 04.12.2000
(51) Int. Cl.: G01N 33/68

(54) **DIRECT SCREENING METHOD**
DIREKTES SCREENINGVERFAHREN
PROCEDE DE CRIBLAGE DIRECT

(30) Priority: 03.12.1999 GB 9928787
(43) Date of publication of application: 25.09.2002
(73) Proprietor: Domantis Limited, Brentford Middlesex TW8 9GS (GB)
(72) Inventor: HOLT, Lucy Jessica, Trumpington, Cambridge CB2 2JE (GB); DE WILDT, Rudolf, Maria, Theodora, Cambridge CB1 8DN (GB); TOMLINSON, Ian, Great Shelford Cambridge CB2 5LJ (GB)
(74) Representative: Maschio, Antonio
(86) International application number: PCT/GB2000/004638
(87) International publication number: WO 2001/040803

(56) References cited:
- WO-A-99/39210
- WO-A-99/41383
- DE WILDT RUUD M T ET AL: "Antibody arrays for high-throughput screening of antibody-antigen interactions" NATURE BIOTECHNOLOGY,NATURE PUBLISHING,US, vol. 18, no. 9, September 2000 (2000-09), pages 989-994, XP002162316 ISSN: 1087-0156
- HOOGENBOOM H R ET AL: "Antibody phage display technology and its applications" IMMUNOTECHNOLOGY,NL,ELSEVIER SCIENCE PUBLISHERS BV, vol. 4, no. 1, 1 June 1998 (1998-06-01), pages 1-20, XP004127382 ISSN: 1380-2933

## Description

The present invention relates to a method for screening repertoires of polypeptides whereby the polypeptides are translated in close proximity to a target molecule or molecules, such that members of the repertoire that interact with the target molecule or molecules can be identified. In particular, the invention relates to a method for expressing a repertoire of polypeptides *in situ* in an array form, and detecting the interaction thereof with immobilised target molecules.

### Introduction

Mass screening of antibody fragments for antigen binding was first described using lytic plaques (Huse *et al..* 1989; Mullinax *et al.,* 1990). These libraries were made from immunised mice or immune-boosted individuals, which has the disadvantage that for each target antigen a new library has to be generated and no reproducible duplicate filters can be made. Therefore, phage display libraries of antibody fragments have been constructed from naive (non-immunised) donors (Marks et al. (1991) J. Mol. Biol., 222: 581: Vaughan et al. (1996) Nature Biotech., 14: 309; Sheets. M. D., Amersdorfer, P., Finnern, R., Sargent. P., Lindqvist. E., Schier, R., Hemingsen, G., Wong, C., Gerhart. J. C. & Marks, J. D. (1998). Efficient construction of a large nonimmune phage antibody library: the production of high-affinity human single-chain antibodies to protein antigens. Proc Natl Acad Sci USA 95, 6157-62) or from cloned variable (V) region genes with synthetically introduced complementarity determining regions (CDRs) (Griffiths et al. (1994) EMBO J., 13: 3245) which should contain a huge variety of antibodies. Indeed, after repeated rounds of selection and affinity purification on antigen, antibody fragments against a range of antigens have been isolated, that were previously considered to be difficult, such as self-antigens (Griffiths, A. D., Malmqvist, M.. Marks, J. D., Bye, J. M., Embleton, M. J., McCafferty. J., Baier, M., Holliger, K. P., Gorick, B. D., Hughes Jones, N. C. & et al. (1993). Human anti-self antibodies with high specificity from phage display libraries. EMBO J. 12, 725-734; Griffiths et al., 1994; De Wildt, R. M. T., Finnern, R., Ouwehand, W. H., Griffiths, A. D., Van Venrooij, W. J. & Hoet, R. M. A. (1996). Characterization of human variable domain antibody fragments against the U1 RNA-associated A protein, selected from a synthetic and a patient-derived combinatorial V gene library. Eur.J.Immunol. 26, 629-639) and MHC-peptide complexes (Andersen *et al.,* 1996).

The experimental procedure of phage display involves repeated rounds of phage growth, panning and infection, which often result in biases towards immune-dominant epitopes and dominant proteins (in protein mixtures). It is therefore useful to analyse clones as early as possible in order to retain most of the diversity in potential binding clones.

This requires a mass screen for antibody binding to antigen. Whilst this may be achieved by growing and inducing recombinant antibodies in a 96 well format and then performing a conventional ELISA by transferring the expressed antibodies to another plate coatedwith antigen, it is impracticle to screen more than a 1000 different antibodies in this way. Larger screens may be achieved by robotic pipetting of individual expressed antibodies, nevertheless, the antibodies would still need to compartmentalised during induction which limits the number of antibodies that can be screened with existing plate format and current limitations to the speed of liquid handling. In order to acheive larger screens in the 10³-10⁵ range, antibody containing bacteria can been expressed *in situ,* and then captured on filters for subsequent probing with labelled antigen. Skerra et al., Anal.Biochem. 196, 151-155 used a two-filter approach to capture expressed antibody fragments in order to discriminate between binding and non-binding antibodies - the use of a second filter reducing the background due to bacterial debris. Capturing reagents have also been used to capture antibody fragments expressed in plaques of λ phages (Watkins. J. D., Beuerlein, G., Wu, H., McFadden, P. R., Pancook, J. D. & Huse, W. D. (1998). Discovery of human antibodies to cell surface antigens by capture lift screening of phase-expressed antibody libraries. Anal Biochem 256, 169-77). Both of these techniques rely on the indiscriminate capture of antibodies, followed by the use of a labelled antigen to detect antigen-specific clones. Not only does this require the labelling of each antigen to be screened but the use of low affinity generic capturing reagents may mean that the antibodies come away from the filter before the specific interaction is able to be detected.

To counter these problems we have devised a new approach whereby the target molecule itself is immobilised on the solid support and the polypeptide repertoire is expressed in close proximity, such that binding polypeptides can be directly captured on the solid support containing the target molecule. Furthermore, we have devised a system whereby duplicate filters containing the same repertoire of polypeptides can be produced and screened with two or more different target molecules, such that polypeptides that interact with individual target molecules, with some but not all target molecules, with all target molecules or with none of the target molecules can be identified. In addition to being used to screening for binding interactions this direct expression/interaction assay can be used for any biological screens that involve polypeptides with a biological function. In our approach nucleic acid molecules encoding the polypeptides repertoire and the immobilised target molecules are juxtaposed prior to expression of the nucleic acid molecules and subsequent detection of polypeptide target interactions. Other existing high-density screening methods, such as WO 99/39210 describe the expression of the nucleic acid molecules after the library array has been formed.

### Summary of the Invention

We have developed a methodology by which a repertoire of polypeptides, including antibody polypeptides, can be used to screen for reactivity with one or more target molecules. We have combined polypeptide expression and interaction screening, and created an array of polypeptides which correspond with arrayed nucleic acids encoding the polypeptides. This has the advantage over previous techniques that the expressed polypeptide is able to interact directly with the target molecule rather than being expressed and stored *or* captured for later screening with the target molecule. The production of such arrays also enables the parallel screening of the same repertoire members against different target molecules.

In a first aspect of the present invention, therefore, there is provided a method for screening a repertoire of polypeptides to identify one or more members thereof which interact with one or more target molecules, comprising:
a) immobilising the target molecule(s) on a support;
b) arraying a plurality of nucleic acid molecules encoding the repertoire of polypeptides;
c) juxtaposing the target molecule(s) and the arrayed nucleic acid molecules;
d) expressing the arrayed nucleic acid molecules to produce the polypeptides such that said polypeptides come into contact with the target molecule(s) on the support and a subset of the polypeptides interact with the target molecules; and
e) detecting the interaction of the polypeptides with the target molecules on the support,

wherein the nucleic acid molecules encoding the repertoire of polypeptides and the immobilised target molecules are arrayed and the supports are juxtaposed prior to expression of the arrayed nucleic acid molecules.

The invention incorporates the key advantage of phage display and other expression-display techniques, namely that the nucleic acids encoding the members of a polypeptide repertoire are associated with the individual polypeptide encoded thereby and can thus be selected on the basis of the functional characteristics of the individual polypeptide. Unlike phage display, however, in which this association is achieved by linking the nucleic acids and the polypeptides using bacteriophage which display the polypeptides on the outside and contain the corresponding nucleotide precursor on the inside, the subject invention exploits a novel arraying technique to provide this association. By eliminating the requirement for the nucleic acids and the polypeptides to be retained in or on bacterial cells or to be compartmentalised during expression (for example, in 96 wells plates or using other compartmentalisation strategies) the present invention enables large numbers of polypeptides to be screened simultaneously. Thus, the nucleic acid, its corresponding polypeptide product and the interaction of this polypeptide product with a target molecule(s) are all located in close proximity. If a particular interaction is observed, the corresponding nucleic acid member can be easily identified. Thus, the invention may be extended beyond selection of binding activities to select any polypeptide repertoire on the basis of any functional property of the polypeptides, including enzymatic activity, conformation or any other detectable characteristic.

The target molecules are immobilised onto a solid-phase support, such as for example a membrane filter support, and juxtaposed to the array of nucleic acid molecules. This may involve arraying just the target molecule, such as when coating the support with a purified protein or the target molecule in a complex mixture of other molecules, for example, whole cells or a cell extract. Indeed, for certain screens the precise nature of the target molecule may not be known in which case any polypeptide which produces an interaction during the screen can be used to further characterise the target molecule. Since the polypeptides produced by expression of the nucleic acid molecules are juxtaposed to the target molecules, they have the potential to interact with them. The interaction may be detected using suitable detection systems, which will be chosen on the basis of the interaction being detected.

Detection of the interaction between the polypeptides of the repertoire and the target molecules may be performed in a number of ways, depending on the nature of the interaction itself. For example, if the polypeptide repertoire is an antibody molecule repertoire and the target molecules are antigens, binding between the antibodies and the antigens may be detected by probing the target molecule support using a suitable labelled anti-immunoglobulin, or a labelled superantigen such as Protein A or Protein L. In an alternative embodiment, the target molecules used to select the polypeptide repertoire may be capable of generic interaction with correctly folded and expressed polypeptides. For example, if the target molecule is Protein A or Protein L, the subset of all functional immunoglobulin molecules would be selected form an immunoglobulin molecule repertoire. Alternatively the interaction between the polypeptides of the repertoire and the target molecules may be detected by virtue of some physical change in the nature of the target molecule or other molecules which which they are able to interact. Thus, for example, if the target molecule is a receptor which triggers apoptosis and cells containing the target molecule have been immobilised on the solid support, a interacting member of the polypeptide repertoire may kill the cell, which can be detected under a microscope or by using some other molecular marker of apoptosis. Alternatively, the interacting member of the polypeptide repertoire might induce a colour change in the target molecule.

In a specific embodiment, the invention comprises a method as defined above and according to claims 1 and 18 for screening a repertoire of antibody polypeptides to identify one or more members thereof which bind to one or more target molecules, comprising:
a) immobilising the target molecule(s) on a first support;
b) transforming a plurality of cells with nucleic acid molecules encoding the repertoire of antibody polypeptides and arraying the bacterial cells on a second support:
c) juxtaposing the first and second supports;
d) expressing the nucleic acid molecules to produce the antibody polypeptides such that said polypeptides are secreted from the cells on the second support and come into contact with the target molecule(s) on the first support and a subset of the polypeptides binds to the target molecules; and
e) detecting the binding of the antibody polypeptides with the target molecules on the first support

The cells may be prokaryotic or eukaryotic, including bacterial cells such as *E. coli,* lower eukaryotic cells such as yeast cells, and higher eukaryotic cells such as mammalian cells. In the subject embodiment of the invention, arraying is advantageously performed by robotic colony picking into culture plates, from which many duplicate filters may be produced. Although the first and second filters may be the same, such that a target molecule is first attached to the filter and then the bacteria are arrayed, grown and expressed on the a target molecule filter, it is advantageous to place the second filter comprising the arrayed colonies on top of a first filter having immobilised thereon the target molecules, such that the first filter is in contact with the underside of the second filter, and not in contact with the colonies themselves.

Accordingly, the first and second supports which may be used in the methods according to the present invention are advantageously filters. At least the second filter is advantageously a porous or microporous filter, which allows the polypeptides secreted by the cells to pass therethrough and make contact with the first filter. Preferred filter materials include nitrocellulose, PVDF and other artificial membranes known in the art.

The polypeptides expressed and secreted by the cells are allowed to pass through the second filter, and interact with the immobilised target molecules on the first filter by binding thereto. Bound immunoglobulins may be detected using anti-immunoglobulin reagents, such as labelled superantigens.

### Brief Description of the Drawings

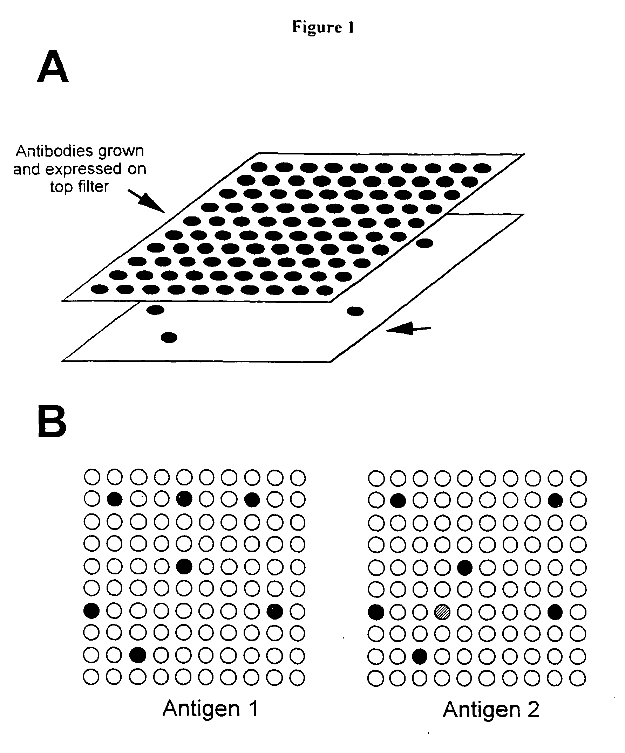

**Figure 1****. Outline of method to screen bacterial expressed scFvs.**
   (A) Bacteria containing cloned antibody genes are arrayed onto a filter and grown overnight in the absence of scFv production. A filter coated with the target antigen is prepared and placed onto a second plate that contains IPTG for scFv induction, which is then covered with the filter containing the gridded bacteria. During induction, antibodies will pass through the top filter and if antigen specific will bind the antigen on the bottom filter. After several hours induction the bottom filter is removed and bound scFvs are detected using a secondary reagent. (B). By applying robotic picking and gridding duplicate ordered arrays can be produced which can be screened against two or more different target molecules to identify members of the repertoire that bind (in this example) Antigen 1 and Antigen 2 (black circles), Antigen 1 but not Antigen 2 (shaded circles), Antigen 2 but not Antigen 1 (hatched circles), neither Antigen (white circles).

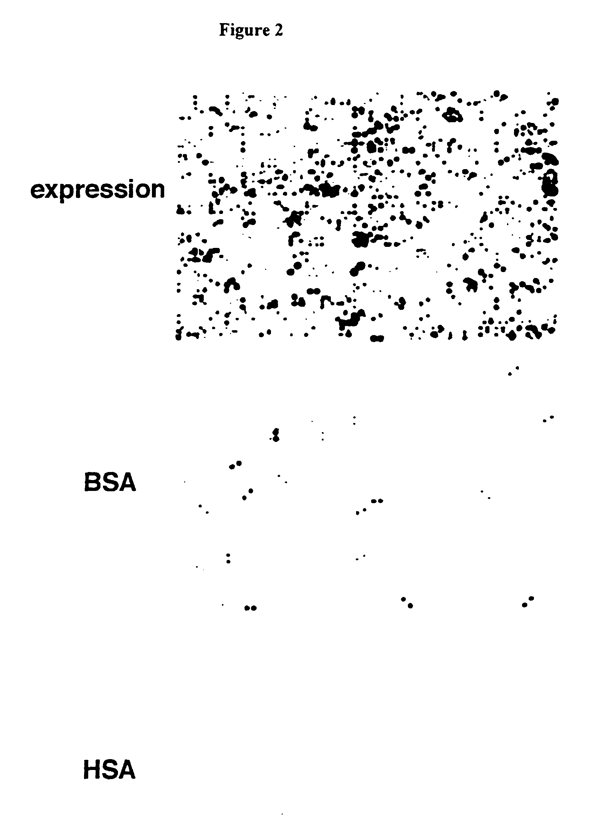

**Figure 2****. Analysis of BSA-specific scFvs on a macro-array**
   A) Sandwich detection of functional scFvs using Protein L as a the target molecule and protein A-HRP as a detection reagent B) Screening for BSA-binding scFvs using immobilised BSA to bind BSA-binding scFvs C) Screening for HSA-binding scfvs scFvs using immobilised HSA to bind HSA-binding scFvs. The panel depicted here consists of 6144 clones. Clones were arrayed in a 4x4 pattern of duplicate clones from 8 384-well microtitre plates (384x8x2).

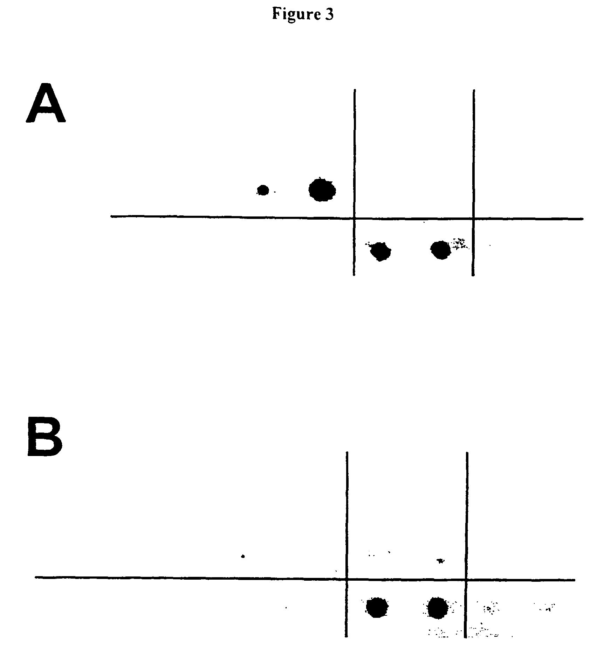

**Figure 3****. Filter screening for interacting protein pairs**
   (A) detection of anti-M scFv-antigen M and anti-T scFv-antigen M pair (B) detection of FRB-FKBP12 pair. Interacting pairs are detected by capturing expressed GST-fusions with an anti-GST antibody and the interacting ligand is detected using protein L HRP. The FRB-FKBP 12 interaction was only observed when rapamycin was present.

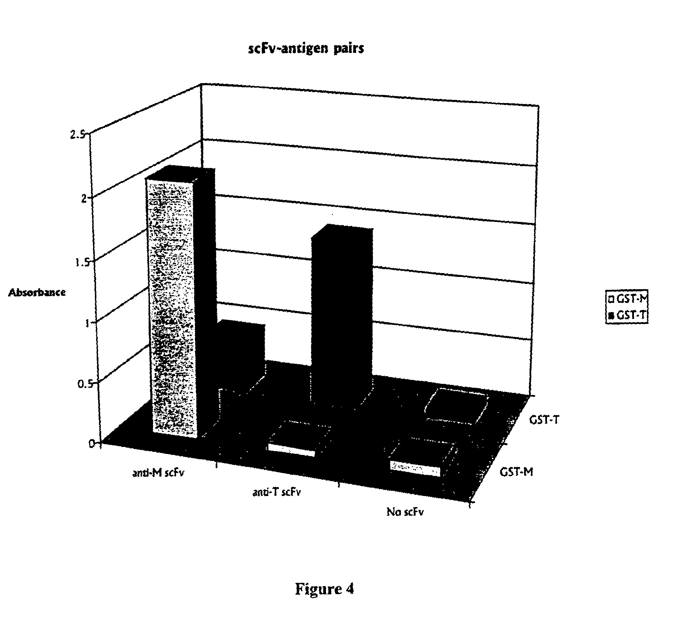

**Figure 4****. ELISA detection of interacting protein pair**
   (A) detection of scFv-antigen M and scFv-antigen T pair (B) detection of FRB-FKBP12 pair. Interacting pairs are detected by capturing expressed GST-fusions with an anti-GST antibody and the interacting ligand is detected using protein L HRP. The FRB-FKBP12 interaction was only observed when rapamycin was present.

### Detailed Description of the Invention

### Definitions

**Array** An array as referred to herein, is any spatial arrangement of nucleic acid members of the repertoire whereby different nucleic acid members are arranged at discrete and predefined positions on a solid support. Such ordered arrays may be created using a gridding technique, such as robotic picking, to arrange the members into desired positions. Arrays of this type have the advantage that each clone has its unique position and multiple duplicate filters can be generated and screened against different target molecules. Further preferred arraying technologies are further described below.

The nucleic acid molecules according to the invention may be arrayed in any desired form. For example, they may be arrayed as naked nucleic acids, either RNA, DNA or any other form of nucleic acid. In a preferred aspect of the invention, however, they are arrayed in the form of cells, or aggregates of cells, transformed with the nucleic acids. Suitable cells include bacterial cells, eukaryotic cells and higher eukaryote cells such as mammalian cells.

Preferably, the nucleic acid molecules are in the form of expression vectors which encode the members of the repertoire of polypeptides operatively linked to control sequences sufficient to direct their transcription and/or translation. For example, such control sequences may include promoter sequences and enhancers, as are known to those skilled in the art, which are necessary for the transcription of DNA molecules.

For example, the nucleic acid molecules may be in the form of plasmids, viruses, bacteriophage, linear nucleic acid molecules whether in naked or complexed form. They are then transcribed (if DNA based) and/or translated, to produce the polypeptides, *in situ* on the array.

An array may comprise any suitable number of members, for example 10. 100 or 500 members. Preferably, an array to be used in the methods according to the invention comprises at least 1000 members, advantageously 10⁴, 10⁵, 10⁶ or more members.

**Generic ligand** A generic ligand is a ligand that binds a substantial proportion of functional members in a given repertoire of polypeptides. Thus, the same generic ligand can bind many members of the repertoire regardless of their target ligand specificities (see below). In general, the presence of functional generic ligand binding site indicates that the repertoire member is expressed and folded correctly. Thus, binding of the generic ligand to its binding site provides a method for preselecting functional polypeptides from a repertoire of polypeptides. Target molecules may also have generic ligands that can be used to indicate the functionality of the target molecules.

**Interact** In the context of the present invention, "interact" refers to any detectable interaction between the polypeptides and the target molecules. For example, in the case of antibody polypeptides, such as scFv, the interaction may be a binding interaction and the target polypeptides may be antigens. Alternatively, the interaction may be an enzymatically-catalysed reaction, in which the polypeptides may be enzymes and the target molecules substrates therefor. Frequently, binding of polypeptides such as enzymes or molecules involved in cell signalling involves a binding event and a change in a measurable biochemical activity, such as a kinase activity or a phosphatase activity. Such activities are measurable using standard assay methodologies known in the art.

**Juxtaposition** In the context of the present invention, "juxtaposition" includes but is not limited to physical contact. The repertoire and the target molecules are juxtaposed such that the polypeptides expressed on the array are capable of interacting with the target molecules on the support, in such a manner that the site of interaction of each individual member of the repertoire with the target molecule can be correlated with its position on the array. In a preferred aspect, the support having the target molecules immobilised thereon is placed in contact with the support on which the nucleic acids encoding the repertoire of polypeptides are arrayed.

**Polypeptide** The term "polypeptide" refers to any kind of polypeptide such as peptides, human proteins, fragments of human proteins, proteins or fragments of proteins from non-human sources, engineered versions proteins or fragments of proteins, enzymes, antigens, drugs, molecules involved in cell signalling, such as receptor molecules, antibodies, including polypeptides of the immunoglobulin superfamily, such as antibody polypeptides or T-cell receptor polypeptides. According to the present invention all such "polypeptides" are capable (or potentially capable) of an interaction with a target molecule, which in the case of a binding interaction would be a target ligand.

Advantageously, the antibody polpeptides may comprise both heavy chain (V_{H}) and light chain (V_{L}) polypeptides, or single domain antibody repertoires comprising either heavy chain (V_{H}) aor light chain (V_{L}) polypeptides. An antibody polypeptide, as used herein, is a polypeptide which either is an antibody or is a part of an antibody, modified or unmodified. Thus, the term antibody polypeptide includes a heavy chain, a light chain, a heavy chain-light chain dimer, a Fab fragment, a F(ab')₂ fragment, a heavy chain single domain, a light chain single domain, a Dab fragment, or an Fv fragment, including a single chain Fv (seFv). Methods for the construction of such antibody molecules and nucleic acids encoding them are well known in the art.

**Repertoire** A "repertoire" is a population of diverse variants, for example nucleic acid variants which differ in nucleotide sequence or polypeptide variants which differ in amino acid sequence. Generally, a repertoire includes a large number of variants, sometimes as many as 10¹⁰, 10¹¹, 10¹² or more. Large repertoires comprise the highest number of possible variants for selection. Smaller repertoires may be constructed and are extremely useful, particularly if they have been pre-selected to remove unwanted members, such as those including stop codons, incapable of correct folding or which are otherwise inactive. Such smaller repertories may comprise 10. 10², 10³. 10⁴, 10⁵, 10⁶ or more nucleic acids or polypeptides. Advantageously, smaller repertoires comprise between 10² and 10⁵ nucleic acids or polypeptides. According to the present invention, a repertoire of nucleotides is preferably designed to encode a corresponding repertoire of polypeptides.

**Subset** A "subset" is a part of the repertoire. In the terms of the present invention, only a subset of the repertoire is capable of interacting with the target molecule, and thus only a subset of the repertoire will give rise to a detectable interaction on the array. For example, where the target molecule is a specific ligand for an antibody, a subset of antibodies capable of binding to the target ligand is isolated.

**Target molecule** A target molecule is a molecule for which an interaction with one or more members of the repertoire is sought. Thus, the term "target molecule" includes antigens, antibodies, enzymes, substrates for enzymes, lipids, any molecule expressed in or on any cell or cellular organism, any organic or inorganic small molecules, and any other molecules capable of interacting with a member of the polypeptide repertoire. The target molecules may themselves be polypeptides, in which case both the repertoire and the targets are polypeptides. In such a case, the repertoire may be a repertoire of antigens, or substrates for enzymes, which is to be screened against one or more antibodies or enzyme molecules; or *vice versa.* Where the interaction is a binding interaction the target molecule may be a target ligand (see below).

**Target ligand** The target ligand is a molecule for which members of the polypeptide repertoire that have a specific binding activity are to be identified. Where the members of the polypeptide repertoire are antibody molecules, the target ligand may be an antigen and where the members of the repertoire are enzymes, the target ligand may be a substrate. Where the members of the polypeptide repertoire are expressed cDNAs, the target ligands may themselves be antibodies or some other polypeptide molecule.

### Construction of Nucleic Acids encoding Polypeptide Repertoires

In general, nucleic acid libraries encoding repertoires of polypeptides according to the present invention may be constructed using methods analogous to those used to construct libraries for phage display. Such methods are well known in the art (McCafferty et al. (1990) Nature, 348: 552: Kang et al. (1991) Proc. Natl. Acad. Sci. U.S.A.. 88: 4363; Clackson et al. (1991) Nature, 352: 624; Lowman et al. (1991) Biochemistry. 30: 10832; Burton et al. (1991) Proc. Natl. Acad. Sci U.S.A., 88: 10134: Hoogenboom et al. (1991) Nucleic Acids Res., 19: 4133; Chang et al. (1991) J. Immunol., 147: 3610; Breitling et al. (1991) Gene. 104: 147: Marks *et al.* (1991) supra; Barbas *et al.* (1992) supra: Hawkins and Winter (1992) J. Immunol.. 22: 867; Marks et al., 1992, J. Biol. Chem., 267: 16007; Lerner et al. (1992) Science, 258: 1313).

One particularly advantageous approach has been the construction of scFv phage-libraries (Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al.* (1990) supra; Clackson *et al.* (1991) supra; Marks *et al.* (1991) supra; Chiswell et al. (1992) Trends Biotech., 10: 80; Marks *et al.* (1992) supra).

Libraries according to the present invention may advantageously be designed to be based on a predetermined main chain conformation. Such libraries may be constructed as described in International Patent Application WO 99/20749.

Unlike the approaches used for phage display, however, libraries of nucleic acids according to the invention are not constructed as fusions with a phage coat protein gene, and not necessarily constructed in bacteriophage vectors. Where a phage or phagemid vector is used, the polypeptide is advantageously not fused to the coat protein, but separate therefrom. For example, a stop codon may be introduced into the sequence to ensure that the polypeptides are not expressed as a fusion. Any vector capable of expressing a recombinant polypeptide therein is suitable. The polypeptides are advantageously secreted from host cells.

In general, the nucleic acid molecules and vector constructs required for the performance of the present invention are available in the art and may be constructed and manipulated as set forth in standard laboratory manuals, such as Sambrook et al. (1989) Molecular Cloning: A Laboratory Manual, Cold Spring Harbor, USA.

The manipulation of nucleic acids in the present invention is typically carried out in recombinant vectors. As used herein, vector refers to a discrete element that is used to introduce heterologous DNA into cells for the expression and/or replication thereof. Methods by which to select or construct and, subsequently, use such vectors are well known to one of moderate skill in the art. Numerous vectors are publicly available, including bacterial plasmids, bacteriophage, artificial chromosomes and episomal vectors. Such vectors may be used for simple cloning and mutagenesis: alternatively, as is typical of vectors in which repertoire (or pre-repertoire) members of the invention are carried, a gene expression vector is employed. A vector of use according to the invention may be selected to accommodate a polypeptide coding sequence of a desired size, typically from 0.25 kilobase (kb) to 40 kb in length. A suitable host cell is transformed with the vector after *in vitro* cloning manipulations. Each vector contains various functional components, which generally include a cloning (or "polylinker") site, an origin of replication and at least one selectable marker gene. If given vector is an expression vector, it additionally possesses one or more of the following: enhancer element, promoter, transcription termination and signal sequences, each positioned in the vicinity of the cloning site, such that they are operatively linked to the gene encoding a polypeptide repertoire member according to the invention.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA. such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins. e.g. ampicillin, neomycin, methotrexate or tetracycline, complement auxotrophic deficiencies, or supply critical nutrients not available in the growth media.

Since the replication of vectors according to the present invention is most conveniently performed in *E. coli,* an *E. coli*-selectable marker, for example, the β-lactamase gene that confers resistance to the antibiotic ampicillin, is of use. These can be obtained from *E. coli* plasmids, such as pBR322 or a pUC plasmid such as pUC 18 or pUC 19.

Expression vectors usually contain a promoter that is recognised by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Delgarno sequence operably linked to the coding sequence.

In the library of nucleic acid molecules to be used in the methods according to the present invention, preferred vectors are expression vectors that enables the expression of a nucleotide sequence encoding a member of the polypeptide repertoire. Construction of such vectors employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis. Western blotting, dot blotting of DNA, RNA or protein, *in situ* hybridisation, immunocytochemistry or sequence analysis of nucleic acid or protein molecules. Those skilled in the art will readily envisage how these methods may be modified, if desired.

### Mutagenesis using the polymerase chain reaction (PCR)

Once a vector system is chosen and one or more nucleic acid sequences encoding members of the polypeptide repertoire are cloned into the vector, one may generate diversity within the cloned molecules by undertaking mutagenesis prior to expression. Mutagenesis of nucleic acid sequences encoding polypeptide repertoires is carried out by standard molecular methods. Of particular use is the polymerase chain reaction, or PCR, (Mullis and Faloona (1987) Methods Enzymol., 155: 335). PCR. which uses multiple cycles of DNA replication catalysed by a thermostable, DNA-dependent DNA polymerase to amplify the target sequence of interest, is well known in the art.

Oligonucleotide primers useful according to the invention are single-stranded DNA or RNA molecules that hybridise to a nucleic acid template to prime enzymatic synthesis of a second nucleic acid strand. The primer is complementary to a portion of a target molecule present in a pool of nucleic acid molecules used in the preparation of sets of arrays of the invention. It is contemplated that such a molecule is prepared by synthetic methods, either chemical or enzymatic. Alternatively, such a molecule or a fragment thereof is naturally occurring, and is isolated from its natural source or purchased from a commercial supplier. Mutagenic oligonucleotide primers are 15 to 100 nucleotides in length, ideally from 20 to 40 nucleotides, although oligonucleotides of different length are of use.

Typically, selective hybridisation occurs when two nucleic acid sequences are substantially complementary (at least about 65% complementary over a stretch of at least 14 to 25 nucleotides, preferably at least about 75%, more preferably at least about 90% complementary). See Kanehisa (1984) Nucleic Acids Res. 12: 203. As a result, it is expected that a certain degree of mismatch at the priming site is tolerated. Such mismatch may be small, such as a mono-, di- or tri-nucleotide. Alternatively, it may comprise nucleotide loops, which we define as regions in which mismatch encompasses an uninterrupted series of four or more nucleotides.

Overall, five factors influence the efficiency and selectivity of hybridisation of the primer to a second nucleic acid molecule. These factors, which are (i) primer length, (ii) the nucleotide sequence and/or composition, (iii) hybridisation temperature, (iv) buffer chemistry and (v) the potential for steric hindrance in the region to which the primer is required to hybridise, are important considerations when non-random priming sequences are designed.

There is a positive correlation between primer length and both the efficiency and accuracy with which a primer will anneal to a target sequence ; longer sequences have a higher melting temperature (T_{M}) than do shorter ones, and are less likely to be repeated within a given target sequence, thereby minimising promiscuous hybridisation. Primer sequences with a high G-C content or that comprise palindromic sequences tend to self-hybridise, as do their intended target sites, since unimolecular, rather than bimolecular, hybridisation kinetics are generally favoured in solution; at the same time, it is important to design a primer containing sufficient numbers of G-C nucleotide pairings to bind the target sequence tightly, since each such pair is bound by three hydrogen bonds, rather than the two that are found when A and T bases pair. Hybridisation temperature varies inversely with primer annealing efficiency, as does the concentration of organic solvents, e.g. formamide, that might be included in a hybridisation mixture, while increases in salt concentration facilitate binding. Under stringent hybridisation conditions, longer probes hybridise more efficiently than do shorter ones, which are sufficient under more permissive conditions. Stringent hybridisation conditions typically include salt concentrations of less than about 1M. more usually less than about 500 mM and preferably less than about 200 mM. Hybridisation temperatures range from as low as 0°C to greater than 22°C, greater than about 30°C. and (most often) in excess of about 37°C. Longer fragments may require higher hybridisation temperatures for specific hybridisation. As several factors affect the stringency of hybridisation, the combination of parameters is more important than the absolute measure of any one alone.

Primers are designed with these considerations in mind. While estimates of the relative merits of numerous sequences may be made mentally by one of skill in the art, computer programs have been designed to assist in the evaluation of these several parameters and the optimisation of primer sequences. Examples of such programs are "PrimerSelect" of the DNAStar^{™} software package (DNAStar. Inc.; Madison, WI) and OLIGO 4.0 (National Biosciences, Inc.). Once designed, suitable oligonucleotides are prepared by a suitable method, e.g. the phosphoramidite method described by Beaucage and Carruthers (1981) Tetrahedron Lett., 22: 1859) or the triester method according to Matteucci and Caruthers (1981) J. Am. Chem. Soc., 103: 3185, or by other chemical methods using either a commercial automated oligonucleotide synthesiser or VLSIPS^{™} technology.

PCR is performed using template DNA (at least 1fg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionised water to a total volume of 25 µl. Mineral oil is overlaid and the PCR is performed using a programmable thermal cycler.

The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenised, mismatch is required, at least in the first round of synthesis. In attempting to amplify a population of molecules using a mixed pool of mutagenic primers, the loss, under stringent (high-temperature) annealing conditions, of potential mutant products that would only result from low melting temperatures is weighed against the promiscuous annealing of primers to sequences other than the target site. The ability to optimise the stringency of primer annealing conditions is well within the knowledge of one of moderate skill in the art. An annealing temperature of between 30 °C and 72 °C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above: 1-2 minutes), and extension (72°C for 1-5 minutes, depending on the length of the amplified product). Final extension is generally for 4 minutes at 72°C, and may be followed by an indefinite (0-24 hour) step at 4°C.

### Expression of Nucleic Acids

Nucleic acid molecules encoding a repertoire of polypeptides in accordance with the present invention may be expressed according to a number of techniques known in the art. As used herein, "expression" denotes the transcription and/or translation of nucleic acids into protein.

Where the nucleic acids are arrayed in the form of naked nucleic acid, or complexed nucleic acid outside of the environment of a cell, components of an *in vitro* biological system are required to express the nucleic acids. These are selected for the requirements of a specific system from the following; a suitable buffer, an *in vitro* transcription/replication system and/or an *in vitro* translation system containing all the necessary ingredients, enzymes and cofactors, RNA polymerase, nucleotides, nucleic acids (natural or synthetic), transfer RNAs, ribosomes and amino acids, and the substrates of the reaction of interest in order to allow selection of the modified gene product.

A suitable buffer will be one in which all of the desired components of the biological system are active and will therefore depend upon the requirements of each specific reaction system. Buffers suitable for biological reactions are known in the art and recipes provided in various laboratory texts, such as Sambrook *et al.,* 1989.

The *in vitro* translation system will usually comprise a cell extract, typically from bacteria (Zubay, 1973: Zubay, 1980; Lesley *et al.,* 1991: Lesley, 1995), rabbit reticulocytes (Pelham and Jackson, 1976), or wheat germ (Anderson *et al.,* 1983). Many suitable systems are commercially available (for example from Promega) including some which will allow coupled transcription/translation (all the bacterial systems and the reticulocyte and wheat germ TNT^{™} extract systems from Promega). The mixture of amino acids used may include synthetic amino acids if desired, to increase the possible number or variety of proteins produced in the library. This can be accomplished by charging tRNAs with artificial amino acids and using these tRNAs for the *in vitro* translation of the proteins to be selected (Ellman *et al.,* 1991; Benner. 1994; Mendel *et al.,* 1995).

Where the nucleic acids are arrayed in the form of cells or cell colonies, expression takes place within the cell itself. Suitable host cells are known in the art. Host cells such as prokaryote, yeast and higher eukaryote cells may be used for replicating DNA and producing the polypeptide repertoire. Suitable prokaryotes include eubacteria, such as Gram-negative or Gram-positive organisms, such as *E. coli,* e.g. *E. coli* K-12 strains, DH5α and HB101, or Bacilli. Further hosts suitable for the polypeptide repertoire-encoding vectors include eukaryotic microbes such as filamentous fungi or yeast, e.g. Saccharomyces cerevisiae. Higher eukaryotic cells include insect and vertebrate cells, particularly mammalian cells, including human cells, or nucleated cells from other multicellular organisms. The propagation of vertebrate cells in culture (tissue culture) is a routine procedure. Examples of useful mammalian host cell lines are epithelial or fibroblastic cell lines such as Chinese hamster ovary (CHO) cells, NIH 3T3 cells, HeLa cells or 293T cells. The host cells referred to in this disclosure comprise cells in in vitro culture as well as cells that are within a host animal.

DNA may be stably incorporated into cells or may be transiently expressed using methods known in the art. Stably transfected mammalian cells may be prepared by transfecting cells with an expression vector having a selectable marker gene, and growing the transfected cells under conditions selective for cells expressing the marker gene. To prepare transient transfectants, mammalian cells are transfected with a reporter gene to monitor transfection efficiency.

To produce such stably or transiently transfected cells, the cells should be transfected with a sufficient amount of the nucleic acid. The precise amounts of DNA encoding the members of the polypeptide repertoire which is required may be empirically determined and optimised for a particular cell and assay.

Host cells are transfected or transformed with the above expression or cloning vectors of this invention and cultured in conventional nutrient media modified as appropriate for inducing promoters, selecting transformants, or amplifying the genes encoding the desired sequences. Heterologous DNA may be introduced into host cells by any method known in the art, such as transfection with a vector encoding a heterologous DNA by the calcium phosphate coprecipitation technique or by electroporation. Numerous methods of transfection are known to the skilled worker in the field. Successful transfection is generally recognised when any indication of the operation of this vector occurs in the host cell. Transformation is achieved using standard techniques appropriate to the particular host cells used.

Incorporation of cloned DNA into a suitable expression vector, transfection of eukaryotic cells with a plasmid vector or a combination of plasmid vectors, each encoding one or more distinct genes or with linear DNA. and selection of transfected cells are well known in the art (see. e.g. Sambrook et al. (1989) Molecular Cloning: A Laboratory Manual. Second Edition, Cold Spring Harbor Laboratory Press).

Transfected or transformed cells are cultured using media and culturing methods known in the art, preferably under conditions whereby the polypeptide repertoire encoded by the nucleic acid molecules is expressed. The composition of suitable media is known to those in the art, so that they can be readily prepared. Suitable culturing media are also commercially available.

### Arraying of Nucleic Acids

Nucleic acids may be arrayed by any one of a variety of methods, depending upon whether the nucleic acids are arrayed as such or contained within cells.

### (a) Synthesis of nucleic acid arrays

Arrays of nucleic acids may be prepared by direct chemical synthesis of nucleic acid molecules. Chemical synthesis involves the synthesis of arrays of nucleic acids on a surface in a manner that places each distinct nucleic acid (e.g.. unique nucleic acid sequence) at a discrete, predefined location in the array. The identity of each nucleic acid is determined by its spatial location in the array. These methods are adapted from those described in U.S. Patent No. 5.143.854: WO90/15070 and WO92/10092; Fodor et al. (1991) Science, 251: 767; Dower and Fodor (1991) Ann. Rep. Med. Chem., 26: 271.

### (b) Arraying of cells

In a preferred aspect of the invention, arrays of nucleic acids may be prepared by arraying cells. Although cells can simply be spread and grown on a filter placed upon bacterial growth media, cells are advantageously arrayed by robotic picking, since robotic techniques allow the most precise and condensed gridding of cell colonies; however, any technique, including manual techniques, which is suitable for locating cells or colonies of cells at discrete locations on a support, may be used.

The gridding of cells may be regular, such that each colony is at a given distance from the next, or random. If colonies are spaced randomly, their density can be adjusted to statistically reduce or eliminate the probability of colonies overlapping on the chosen support.

Methods for arraying cell colonies are described in detail in US patent 5,326,691.

### (c) Arraying of antibodies

Antibodies may be arrayed by robotic gridding using commercial technology as is commonly available in the art, or may be expressed *in situ* from arrayed antibody-producing cells, arrayed for example as described above. Robotic arraying is well-known in the art, and machines are available from companies such as Genetix, Genetic MicroSystems and BioRobotics which are capable of arraying at high speed with great accuracy over small or large surfaces. Such machines are capable of spotting purified protein, supernatant or cells onto porous or non-porous surfaces, such that they can subsequently be fixed thereto if necessary to produce stable arrays. Arrays can be replicated if required, to allow simultaneous screening with multiple target ligands. Cell-based arrays can be lysed to release polypeptides *in situ,* and/or expressed polypeptides can be fixed to the solid support according to known procedures.

### Selection of Polypeptides bound to Target Molecules

As set forth above, the target molecules may be capable of specific interaction with the members of the polypeptide repertoire, or they may be capable of generic interaction. Target molecules capable of specific interaction will interact only with those polypeptides having a desired activity to be selected from the repertoire. Such target molecules include specific antigens for antibodies, or substrates for enzymes. Target molecules capable of generic interactions will generally interact with a somewhat larger subset of the repertoire that has a desired characteristic, for example functional members which are correctly folded or otherwise theoretically capable of functioning. The use, in particular, of specific and generic ligands for antibody polypeptides is described in detail in International Patent Application WO 99/20749.

Once antibody polypeptides have bound to target molecules, they may be detected by the use of different generic ligands, according to the nature of the target molecule used. Thus, if the target molecule is an antigen, labelled Protein L can be used for detection of specific binding members of the repertoire, whereas if the target molecule is Protein L. labelled Protein Acan be used for detection of functional members of the repertoire.

Generic ligands can take the form of superantigens, such as Protein A or Protein L. or suitable antibody molecules. If an appropriate antibody is not publicly available, it may be produced by phage display methodology or as follows.

Either recombinant proteins or those derived from natural sources can be used to generate antibodies using standard techniques, well known to those in the field. For example, the protein (or "immunogen") is administered to challenge a mammal such as a monkey, goat, rabbit or mouse. The resulting antibodies can be collected as polyclonal sera, or antibody-producing cells from the challenged animal can be immortalised (e.g. by fusion with an immortalising fusion partner to produce a hybridoma), which cells then produce monoclonal antibodies.

### a. Polyclonal antibodies

The antigen protein is either used alone or conjugated to a conventional carrier in order to increases its immunogenicity, and an antiserum to the peptide-carrier conjugate is raised in an animal, as described above. Coupling of a peptide to a carrier protein and immunisations may be performed as described (Dymecki et al. (1992) J. Biol. Chem., 267: 4815). The serum is titered against protein antigen by ELISA or alternatively by dot or spot blotting (Boersma and Van Leeuwen (1994) J. Neurosci. Methods, 51: 317). The serum is shown to react strongly with the appropriate peptides by ELISA, for example, following the procedures of Green et al. (1982) Cell, 28: 477.

### b. Monoclonal antibodies

Techniques for preparing monoclonal antibodies are well known, and monoclonal antibodies may be prepared using any candidate antigen, preferably bound to a carrier, as described by Arnheiter et al. (1981) Nature, 294, 278. Monoclonal antibodies are typically obtained from hybridoma tissue cultures or from ascites fluid obtained from animals into which the hybridoma tissue was introduced. Nevertheless, monoclonal antibodies may be described as being "raised against" or "induced by" a protein.

After being raised, monoclonal antibodies are tested for function and specificity by any of a number of means. Similar procedures can also be used to test recombinant antibodies produced by phage display or other *in vitro* selection technologies. Monoclonal antibody-producing hybridomas (or polyclonal sera) can be screened for antibody binding to the immunogen, as well. Particularly preferred immunological tests include enzyme-linked immunoassays (ELISA), immunoblotting and immunoprecipitation (see Voller, (1978) Diagnostic Horizons, 2: 1, Microbiological Associates Quarterly Publication, Walkersville, MD: Voller et al. (1978) J. Clin. Pathol., 31: 507: U.S. Reissue Pat. No. 31,006: UK Patent 2,019,408; Butler (1981) Methods Enzymol., 73: 482: Maggio, E. (ed.), (1980) Enzyme Immunoassay. CRC Press, Boca Raton, FL) or radioimmunoassays (RIA) (Weintraub, B.. Principles of radioimmunoassays, Seventh Training Course on Radioligand Assay Techniques. The Endocrine Society, March 1986, pp. 1-5. 46-49 and 68-78), all to detect binding of the antibody to the immunogen against which it was raised. It will be apparent to one skilled in the art that either the antibody molecule or the immunogen must be labelled to facilitate such detection. Techniques for labelling antibody molecules are well known to those skilled in the art (see Harlour and Lane (1989) Antibodies, Cold Spring Harbor Laboratory, pp. 1-726).

Alternatively, other techniques can be used to detect binding to the immunogen, thereby confirming the integrity of the antibody. These include chromatographic methods such as SDS PAGE, isoelectric focusing, Western blotting, HPLC and capillary electrophoresis.

"Antibodies" are defined herein as constructions using the binding (variable) region of such antibodies, and other antibody modifications. Thus, an antibody useful in the invention may comprise whole antibodies, antibody fragments, polyfunctional antibody aggregates, or in general any substance comprising one or more specific binding sites from an antibody. The antibody fragments may be fragments such as Fv, Fab and F(ab')₂ fragments or any derivatives thereof, such as a single chain Fv fragments. The antibodies or antibody fragments may be non-recombinant, recombinant or humanised. The antibody may be of any immunoglobulin isotype, e.g., IgG, IgM, and so forth. In addition, aggregates, polymers, derivatives and conjugates of immunoglobulins or their fragments can be used where appropriate.

Probes for detecting bound immunoglobulin, whether they be generic ligands or specific antigens, may be labelled according to techniques known in the art. Methods for labelling probes are set forth in, for example, Sambrook, J., Fritsch, E. F. & Maniatis, T. (1989) Molecular Cloning. A Laboratory Manual (Cold Spring Harbor Laboratory Press, N.Y.) or Ausubel, et al., eds. (1990) Current Protocols in Molecular Biology, John Wiley & Sons. Inc.

### Use of polypeptides selected according to the invention

Polypeptides selected according to the method of the present invention may be employed in substantially any process. Where the polypeptides are antibody polypeptides, they may be used in any process which involves ligand-polypeptide binding, including *in vivo* therapeutic and prophylactic applications, *in vitro* and *in vivo* diagnostic applications, *in vitro* assay and reagent applications, and the like. For example, in the case of antibodies, antibody molecules may be used in antibody based assay techniques, such as ELISA techniques, according to methods known to those skilled in the art.

As alluded to above, the molecules selected according to the invention are of use in diagnostic, prophylactic and therapeutic procedures. For example, enzyme variants generated and selected by these methods may be assayed for activity, either *in vitro* or *in vivo* using techniques well known in the art, by which they are incubated with candidate substrate molecules and the conversion of substrate to product is analysed. Selected cell-surface receptors or adhesion molecules might be expressed in cultured cells which are then tested for their ability to respond to biochemical stimuli or for their affinity with other cell types that express cell-surface molecules to which the undiversified adhesion molecule would be expected to bind, respectively. Antibody polypeptides selected according to the invention are of use diagnostically in Western analysis and *in situ* protein detection by standard immunohistochemical procedures; for use in these applications, the antibodies of a selected repertoire may be labelled in accordance with techniques known to the art. In addition, such antibody polypeptides may be used preparatively in affinity chromatography procedures, when complexed to a chromatographic support, such as a resin. All such techniques are well known to one of skill in the art.

Therapeutic and prophylactic uses of proteins selected by the methods according to the invention involve the administration of polypeptides selected according to the invention to a recipient mammal, such as a human. Of particular use in this regard are antibodies, other receptors (including, but not limited to T-cell receptors) and in the case in which an antibody or receptor was used as either a generic or target ligand, proteins which bind to them.

Substantially pure antibodies or binding proteins thereof of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected polypeptides may be used diagnostically or therapeutically (including extracorporeally) or in developing and performing assay procedures, immunofluorescent staining and the like (Lefkovite and Pernis. (1979 and 1981) Immunological Methods, Volumes I and II. Academic Press. NY).

The selected antibodies or binding proteins thereof selected by the methods of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus. Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the antibodies or binding proteins thereof in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton. New York, pp. 179-213; McFarlin et al. (1973) Science, 179: 478: and Satoh et al. (1987) J. Immunol., 138: 179).

The selected antibodies, receptors (including, but not limited to T-cell receptors) or binding proteins thereof selected by the methods of the present invention may also be used in combination with other antibodies, particularly monoclonal antibodies (MAbs) reactive with other markers on human cells responsible for the diseases. For example, suitable T-cell markers can include those grouped into the so-called "Clusters of Differentiation," as named by the First International Leukocyte Differentiation Workshop (Bernhard et al. (1984) Leukocyte Typing, Springer Verlag, NY).

Generally, the present selected antibodies, receptors or binding proteins will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution. Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The selected polypeptides may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the selected antibodies, receptors or binding proteins thereof of the present invention, or even combinations of selected polypeptides according to the present invention having different specificities, such as polypeptides selected using different target ligands, whether or not they are pooled prior to administration.

The route of administration of pharmaceutical compositions may be any of those commonly known to those of ordinary skill in the art. For therapy, including without limitation immunotherapy, the selected antibodies, receptors or binding proteins thereof of the invention can be administered to any patient in accordance with standard techniques. The administration can be by any appropriate mode, including parenterally, intravenously, intramuscularly, intraperitoneally, transdermally, *via* the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

The selected polypeptides can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present selected polypeptides or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of selected antibody, receptor (e.g. a T-cell receptor) or binding protein thereof *per* kilogram of body weight, with doses of 0.05 to 2.0 mg/kg/dose being more commonly used. For prophylactic applications, compositions containing the present selected polypeptides or cocktails thereof may also be administered in similar or slightly lower dosages.

A composition containing a polypeptide selected by a method according to the present invention may be utilised in prophylactic and therapeutic settings to aid in the alteration, inactivation, killing or removal of a select target cell population in a mammal. In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the selected antibodies, cell-surface receptors or binding proteins thereof whereby the undesired cells are killed or otherwise removed from the blood for return to the mammal in accordance with standard techniques.

The invention is further described, for the purpose of illustration only, in the following examples.

### Example 1:

### Selection of scFv Polypeptides

We used a scFv library based on a single fold that was enriched for binding to the target ligand by one round of phage selection either on BSA, a mixture of unpurified recombinant proteins, or a whole HeLa cell lysate. This antibody fold binds to both generic ligands Protein L and A. We used a two-filter method in which bacteria are grown on one filter and the secreted scFvs are captured on another filter. The target molecule on the filter was either the antigen used for selection, another (possibly related) antigen or Protein L. Thus, the screen is for either specific target binding antibodies (in which case detection of bound antibody was using Protein L-HRP) or, in the latter case, functional and well expressed antibodies (in which case detection of bound antibody was using Protein A-HRP). Specific antigen binding molecules representing 0.1-1% of the library, were identified in all cases, with between 5-50% being detected as functional and well expressed as determined using the generic ligansd as a target molecule.

### Methods

### Proteins

Five clones, two with unknown function (C and M), a human chloride ion current inducer protein (D), α-2 globin (H) and ubiquitin (T) were taken from a human brain cDNA library hEX1 (Bussow *et al..* 1998) and expressed as described (Lueking *et al.,* 1999). Crude cell lysates were harvested and used for selection. Expression levels of each clone were determined by transferring serial dilutions of each lysate in a dot-blot apparatus and immobilisation onto a nitro-cellulose membrane. This filter was blocked in 2 % skimmed milk powder PBS (MPBS) for 30 min. and washed three times with PBS. Recombinant protein was detected with anti-RGS-His antibody (Qiagen: 1/2000 in 2% MPBS) followed by anti-mouse HRP antibody (Dako; 1/2000 in 2% MPBS) and developed with chemiluminescent detection reagent (ECL, Amersham). Equimolar amounts of each protein were mixed in each selection and 10, 100 and 1000-fold dilution thereof.

### Phage library

The library we have used is based on a single human framework for V_{H} (V3-23/DP-47 and J_{H}4b) and V_{κ} (O12/O2/DPK9 and J_{κ}l). with side chain diversity (NNK or DVT encoded) incorporated at positions in the antigen binding site that make contacts to antigen in known structures and are highly diverse in the mature repertoire. The fold that is used, is frequently expressed *in vivo,* and it binds to the generic-binding ligands Protein L and A, which facilitate capturing or detection of the scFvs and do not interfere with the antigen-binding site. The libraries have been pre-screened in phagemid/scFv format for binding to Protein A and Protein L so that the majority of clones in the unselected libraries are functional (Tomlinson *et al.,* unpublished results). This library was selected essentially as has been described (Marks et al., 1991), except that KM13 helper phage (contains gene 3 protein with protease cleavage site) was used and phages were eluted with trypsin. This cleaves all gene 3 proteins that have no scFv fusion, which results in the removal of background phage infection and elutes phages with scFv fusion by proteolytic cleavage in the c-myc tag (Kristensen & Winter, 1998). One round of selection was performed on immunotubes (Nunc, Maxisorp) coated with BSA (10 µg/ml PBS), a mixture of five unpurified recombinant proteins (C, D. H. M or T, each at 100 µg/ml or 10-fold dilutions thereof) or a lysate from HeLa cells (100 µg/ml) (Verheijen *et al.,* 1990).

### Gridding/picking

The selected library was plated onto a large square plate (230x 230 mm, Nunc plates containing TYE. 100 µg/ml ampicillin, 1% glucose) at 10⁴ colonies/plate and grown o/n at 30°C. Colonies were picked (BioRobotics colony picker) into 384 well plates (Genetix, containing 2xTY, 100 µg/ml ampicillin, 1% glucose. 8% glycerol) and grown overnight at 37°C. The plates were either directly used or frozen and stored at -70°C. The 384 well plates were gridded in a 4x4 pattern of duplicate clones (Genetix, Q-bot) onto a large square plate (Genetix Q-tray, containing TYE. 100 µg/ml ampicillin. 1% glucose) covered with a nitrocellulose filter (Schleicher & Schuell). Before transfer onto the plate, this filter was blocked in 2% skimmed milk powder PBS (MPBS) for 30 min at room temperature (RT), briefly washed in PBS and soaked in 2xTY. The gridded plates were grown overnight at 37°C. In the meantime another nitrocellulose filter was coated with 0.5 µg/ml Protein L (Actigen), 100 µg/ml bovine serum albumin (BSA), human serum albumin (HSA), bacterial proteins C, D, H, M or T, or HeLa cell proteins (from 10⁷ cells, (Verheijen et al., 1990)) in 100 ml PBS, overnight at 4°C. This filter was blocked in 2% MPBS, for 1 hr RT, washed 3x in PBS. soaked in 2xTY and then transferred onto a large square plate (230x 230 mm, Nunc plates containing TYE, 100 µg/ml ampicillin. 1 mM isopropyl β-D-thiogalactoside (IPTG)). The second filter containing the colonies was transferred onto the plate covered with the first target molecule filter. These plates were incubated for 3 hr at 30°C. A schematic outline of the method is given (Figure 1).

### Probing of filters

The top filter is removed and the bottom filter was washed 3x with PBS/0.05% Tween (PBST) and blocked with 2% MPBS for 30 min at RT. The filters were washed 3x with PBST. In case of the specific antigen binding the filters were incubated with Protein L HRP conjugate (Actigen, 1/4000) in 2% MPBS for 1 hr at RT. In case of the generic assay the filters were processed as described above except that after blocking the filters were incubated with Protein A HRP conjugate (Amersham, 1/5000). The filters were washed 3x with PBST and incubated with streptavidine-HRP (Pierce) 1/5000 in PBST, 15 min at RT. The filters were washed and developed with ECL reagent. All incubations were performed in 50 ml of buffer on a gently agitating shaker.

### ELISA

To determine whether clones identified using the direct capture screen bound BSA in conventional ELISA. 2xTY containing 100 µg/ml ampicillin and 0.1 % glucose was inoculated with 1/100 volume from an overnight culture. These were grown at 37°C until OD₆₀₀ was approximately 0.9. 1 mM IPTG was added and the cultures were shaken at 30°C overnight. Cultures were spun and supernatants were used in ELISA. A 96 well ELISA plate was coated with 10 µg/ml BSA overnight at 4°C. The plates were washed three times with PBS and blocked with 2% Tween/PBS for 2 hour at RT. Plates were washed three times with PBS and 50 µl of supernatant was incubated with 50 µl of 2% Tween/PBS for 1.5 hours at RT. Plates were washed five times with PBST and incubated with Protein L HRP conjugate (1/4000 in 2% Tween PBS) for 1 hour at RT. Plates were washed five times with PBST and reactions were developed with 3,3',5,5'-tetramethylbenzidine and stopped with sulphuric acid.

### Sequencing

PCR products that were amplified with LMB3 (CAG GAA ACA GCT ATG AC) and pHEN seq (CTA TGC GGC CCC ATT CA) and purified using PCR purification columns (Qiagen, CA), were used as a template for sequencing. Sequencing reactions were performed with primers LMB3 for the heavy chains and pHEN seq for the light chains, using an ABI PRISM Big Dye Terminator Cycle Sequencing Kit. (Perkin Elmer, CA). Reactions were run on an automated sequencer (Applied Biosystems 373A, Perkin Elmer).

### Results

In order to obtain a scFv phagemid library that was enriched for binders against the target ligand by one round of phage selection, a library that was selected on one purified antigen (BSA) was used. Using robotics, 8448 clones (22 384-well plates) were picked and gridded, a number that includes almost all the recovered clones present after one round of selection (about 10⁴ phages were eluted). To determine the fraction of clones expressing functional scFvs, scFvs were captured on a Protein L coated filter and were detected with protein A HRP conjugate. This showed that about 50% of the clones were functional and well expressed (Figure 2). In order to identify specifically binding as well as cross-reactive clones, screening was performed on BSA, HSA and a mixture of irrelevant proteins (HeLa cell proteins). BSA, HSA or HeLa cell proteins were coated on the filter and binding scFvs were detected with Protein L conjugate. A typical panel of arrayed scFvs and their reactivity with BSA and HSA is shown in Figure 2B and 2C, respectively. We found that 50 of the 8448 arrayed clones recognise BSA. One of these 50 was found also to recognise HSA and one clone reacted with a protein in HeLa cells (data not shown). A large number of clones that did not recognise BSA or HSA. recognised proteins present in an extract of HeLa cells (data not shown), indicating the selection of 'sticky' clones. Thus, the use of parallel direct screening on different target molecules enables the identification of BSA specific antibodies, antibodies that bind both BSA and HSA that are not broadly cross-reactive, cross-reactive ("sticky") antibodies and antibodies that bind none of the target molecules.

To further confirm whether the BSA positive clones were specific, they were tested in ELISA for binding to BSA. Most of these clones were indeed positive in ELISA and of these, 16 were further characterised by sequencing. Clones that did not recognise BSA in ELISA expressed scFv at low levels or did not have a full-length insert. The positive identification, on the initial screen, of the clones expressing single domains is probably due to the hydrophobic residues that are normally buried in the V_{H}/V_{L} interface. Sequencing of the V_{H} and V_{L} genes of 16 BSA-specific clones showed that five different clones were present (Table 1). Two clones were present multiple times. Clone 29IJ11 was found twice and clone 29IJ7 was found 11 times. Three other clones were seen once. The sequences of the V_{H} CDR3 region of these anti-BSA scFvs show a consensus at the randomised residues. This confirms that these clones are specific since consensus CDR residues are typically found in specific Abs against other antigens (Clackson *et al.,* 1991; De Wildt *et al.,* 1997).

Next we screened a scFv library that was selected on a mixture of five unpurified recombinant proteins C, D, H, M or T or 10-fold dilutions thereof. 12288 clones (32 x 384) were gridded and multiple copies of this library were screened for binding to the five recombinant proteins separately. Potential specific scFv clones on the initial screen were tested in ELISA for binding to the recombinant proteins. Specific scFv were identified for D (15 clones), M (12 clones) and T (20 clones) and some of these scFv were isolated using a 1000-fold dilutions of protein mixture. The sequences of these clones are depicted in Table 1 and the majority of the anti-ubiquitin clones show a consensus sequence in their V_{H} CDR3 residues. Some clones have different nucleotide sequences although they encode the same CDR3 amino acid residues.

Lastly we screened a scFv library that was selected on a lysate of HeLa cells. 18.342 clones were gridded and tested for binding to filters coated with HeLa cell lysate, yeast cell lysate or an irrelevant protein (FITC-BSA). Several potential scFv clones were tested on a Western blot of HeLa cells or Yeast cells. Five clones were found to recognise a protein present in HeLa but not in Yeast cells.

**Table 1. V_{H} and V_{L} CDR sequences from clones selected from a library based on a single human framework. Underlined residues are randomised in the library.**

| **Clone** | **Library¹** | **CDR111** | **CDR112** | **CDR113** | **CDRL1** | **CDRL2** | **CDRL3** | **No.²** | **Mutations³** |
|---|---|---|---|---|---|---|---|---|---|
| 29IJ1 | DVT | SYAMS | NIYASGDSTAYADSVKG | GYYTFDY | RASQSISSYLN | YASELQS | QQAGYSPTT | 1 | H66 CGG (R) →AGG (R) |
| 29IJ6 | NNK | SYAMS | HISPYGANTRYADSVKG | GLRAFDY | RASQSISSYLN | RASLLQS | QQGRDRPAT | 1 | |
| 29IJ7 | DVT | SYAMS | SIYYDGDSTSYADSVKG | SYYTFDY | RASQSISSYLN | AASSLQS | QQNDSSPTT | 11 | |
| 29IJ11 | DVT | SYAMS | SITGNGSYTAYADSVKG | NSGTFDY | RASQSISSYLN | YASDLQS | QQNTAYPTT | 3 | |
| 29IJ12 | DVT | SYAMS | TIYYNGTSTGYADSVKG | GYTTFDY | RASQSISSYLN | SASSLQS | QQNDYGPTT | 1 | |

| | | | | | | | | | |
|---|---|---|---|---|---|---|---|---|---|
| ¹The library (NNK/DVT diversity) the clones were derived from. ²Number of times the same sequence was observed. ³All nucleotide (amino acid) changes different from V_{H} (V3-23/DP-47 and J_{H}4b) and V_{κ} (O12/O2/DPK9 and J_{κ}1) germline genes, that are not at specifically randomised positions. | | | | | | | | | |

In the foregoing example, it is demonstrated that recombinant antibodies can be used for a direct array screen. We used two approaches: The first approach, the coating of the antigen on the filter and the direct detection of only specifically bound scFvs, has the advantage that no biotinylation or tagging of the target molecules is required. This format results in increased sensitivity because it only involves two incubation steps (blocking and detection) and because scFvs tend to dimerise, resulting in the binding of multiple Protein L conjugates. This approach also offers the potential to screen a large number of scFvs simultaneously on one or more antigens, since the bacteria harbouring the corresponding nucleic acid sequences can be duplicate spotted onto the same or different filters: currently 18,342 colonies are double spotted on one macro-array, which is equivalent to 384 96 well ELISA plates. They also allow identification of differential specific scFvs and cross-reactive scFvs, which is shown by the clones that recognise only BSA. both BSA and HSA, and the clones that recognise proteins in HeLa cells but not in yeast. BSA and HSA are more then 75% homologous, it is therefore not surprising that some antibodies recognise epitopes present on both proteins. Furthermore these arrays can be use to screen impure antigens, which is demonstrated by the identification of scFv specific for the recombinant proteins D. M and T. In contrast to other protein arrays (Bussow et al., 1998) these antibody arrays do not require any denaturation-steps to immobilise the proteins. Indeed, the target molecule can be native, denatured, proteolised or pre-treated in any other way prior to coating on the bottom filter.

### Comparative Example 2

### Comparison with Conventional Phage Display

Comparison of our results with those obtained by several rounds of conventional phage display selection shows that three of the scFvs identified here were also isolated after 3-4 rounds of phage selection. However, using by employing the direct array screen after only a single round of phage selection we found two additional clones (clone 29IJ11 and 29IJ12) which were not seen after extensive screening of the r3 and r4 selections. This shows that these are lost by competition between the different clones during the multiple infection, growth and phage production steps involved in 3 or 3 rounds of phage selection. Since only 16 out of 50 positive clones were sequenced, it is likely that more unique BSA-specific scFvs were present in these 50 positives. The ECL signals on the array of these two unique clones are of a comparable intensity as, for example, clone 291J1 that has a nanomolar affinity. This shows that potentially useful scFvs are lost employing multiple rounds of phage display and thus high throughput screens of the type described here are essential for isolating the most diverse collection of binding clones.

Duplicate arrays of antibodies can be used to screen for antibodies that recognise subtle modifications of a given protein (such as single amino acid mutations or post-translational modifications), or to screen for binders against impure antigens such as molecules expressed on cellular surfaces. Multiple rounds of phage selection often result in binders against other immuno-dominant epitopes or other cell surface molecules (Hoogenboom *et al.,* 1999). In order to prevent this, selections against impure antigens have been performed using depletion or subtraction strategies (De Kruif *et al.,* 1995: Cai & Garen. 1996), or a ligand directed technique called "Pathfinder" (Osbourn *et al.,* 1998). The direct high-thoroughput screen described here can be applied to any of these techniques. The screening procedure can also be performed using multiple target ligands. The target ligands may be different proteins mixed with eachother or may be an extract of cellular proteins. Provided that each target protein in a mixture is present and immobilised in sufficient quantities this should allow detection of specific scFv.

The screen can be used to identify higher affinity binders from a library of mutants of a given antibody, created either by chain shuffling (Marks *et al.,* 1992) or CDR diversification (Schier *et al.,* 1996). In order to humanise or create higher affinity antibody fragments, libraries of antibody fragments can be generated by directed or random mutagenesis using oligonucleotides or error prone polymerases. These libraries or parts of these libraries can easily be grown and expressed in array format and subsequently screened for binding or improved binding to their target ligand.

Arrays of recombinant antibodies have useful applications in the characterisation of protein expression including modifications of these proteins, a field known as proteomics. In contrast to genomics, which studies mRNA expression levels through cDNA arrays, proteomics gives a direct handle on protein functionality. This area is currently dominated by 2D electrophoresis. Arrays of recombinant antibodies can be used to complement the existing technologies, to analyse the expression of one or more proteins in a mixture of proteins (e.g. a cell extract).

In general, this method enables high throughput analysis for ligand binding of recombinant antibodies or other recombinant proteins that are expressed in bacterial periplasm. Especially those cases when the hit-rate is too low to detect with conventional ELISA, but high enough to identify with arrays (>10⁻⁴) and when repeated rounds of phage selection are not beneficial. However, the approach is not restricted to antibody-antigen interactions and may be used to study other functional protein-ligand interactions including screening for active enzymes. For example, a library of transcription factor variants or other DNA binding proteins can be tested for their ability to bind a particular DNA sequence. For example, zinc fingers are small DNA-binding modules noted for their occurrence in a large number of eukaryotic transcription factors, and they bind a certain number of DNA sequences. In the past zinc-fingers have been engineered that bind specifically to a unique nine-base-pair region of a BCR-ABL fusion oncogene and it results in blockage of transcription of this oncogene (Choo *et al.,* 1994). Array screening can be useful to identify such sequence specific DNA binding peptides or proteins that can inhibit the production of factors essential for tumor growth.

A strategy for the selection of active catalysts involves the growth and expression of a library of enzymes in array format on one filter, in close proximity to the target ligand that is immobilised on the other filter. Next, converted substrate is detected using specific anti-product affinity reagents on the target ligand filter. Because the enzyme and its substrate are present in close proximity for several hours it is hoped that this enables the detection of enzymes with high turnover rates but also those with lower turnover rates. In this way we can improve existing phage selection technologies for catalysts that are based on product binding, such as for example selection for aldolases (Barbas *et al.,* 1997).

Whole cells can also be used as target ligands in array sreening. For example, an eukaryotic cell line such as HeLa, can be grown and immobilised on filters. A library of adhesion molecules can then be screened for a functional interaction such as the induction of apotosis via the cell surface molecule CD95 (APO-1, FAS). The readout can be a downstream event such as the translocation of phophatidyl serine or the expression of p53. A randomised library of CD95 ligands, which recognise the CD95 receptor, can be screened and assayed by annexin-V detection of cell surface phosphatidyl serine (Boehringer Mannheim) or by p53 detection (Boehringer Nlannheim).

### Example 3

### Detecting interacting protein pairs

To determine whether filter screening can be used to detect two interacting proteins we investigated pairs of proteins that are known to interact. Protein M and an anti-M scFv M12, protein T and an anti T scFv T15 and FKBP12 rapamycin binding (FRB) domain of FKBP-rapamycin-associated-protein (FRAP) and human immunophilin FKBP12 (12 kD FK506 binding domain). The FRB-FKBP12 interaction depends on the presence of the small molecule rapamycin. Genes encoding M. T or FKBP12 were cloned into pACYC (chloramphenicol resistant) as a C-terminal fusion to glutathion-S-transferase (GST). M12 (anti-M scFv) and T15 (anti-T scFv) are cloned into pHEN derivative pIT2 (ampicillin resistant). FRB and FKBP12 were cloned into pIT2 as a C-terminal fusion to a V kappa single domain.

Single colonies were picked from a fresh agar plate are grown o/n at 37°C in liquid culture (TYE, 100 µg/ml ampicillin or 10 µg/ml chloramphenicol, 1 % glucose). Several combinations of these cultures were mixed together (interacting partners or controls) and were spotted onto an agar plate (TYE, 1% glucose) covered with a nitrocellulose filter (Schleicher & Schuell). Before transfer onto the plate, this filter was blocked in 2% skimmed milk powder PBS (MPBS) for 30 min at room temperature (RT), briefly washed in PBS and soaked in 2xTY. The gridded plates were grown overnight at 37°C. In the meantime another nitrocellulose filter was coated with 2 µg/ml anti-GST antibody (Pharmacia Biotech) in PBS, overnight at 4°C. This filter was blocked in 2% MPBS, for 1 hr RT, washed 3x in PBS, soaked in 2xTY and then transferred onto an agar plate (TYE, 1 mM isopropyl β-D-thiogalactoside (IPTG)). For detection of the FRB-FKBP12 interaction 0.1 ng/ml rapamycin or no rapamycin was added to the plates. The second filter containing the bacterial clones was transferred onto the plate covered with the anti-GST coated filter. These plates were incubated for 5 hr at 30°C.

### Probing of filters

The top filter is removed and the bottom filter was washed 3x with PBS/0.05% Tween (PBST) and blocked with 2% MPBS for 30 min at RT. The filters were washed 3x with PBST and incubated with Protein L HRP conjugate (Actigen, 1/4000) in 2% MPBS for 1 hr at RT. The filters were washed and developed with ECL reagent (Amersham). All incubations were performed on a gently agitating shaker.

### ELISA

To confirm whether clones identified using the direct capture screen bound also in ELISA. 2xTY containing 100 µg/ml ampicillin or 10 µg/ml chloramphenicol and 0.1 % glucose was inoculated with 1/100 volume from an overnight culture. These were grown at 37°C until OD₆₀₀ was approximately 0.9. 1 mM IPTG was added and the cultures were shaken at 25°C or 30°C (scFvs) overnight. Cultures were spun and supernatants were used in ELISA. A 96 well ELISA plate was coated with anti-GST. 2 µg/ml in PBS overnight at 4°C. The plates were washed three times with PBS and blocked with 2% Tween/PBS (for scFv-antigen pairs) or 2%BSA/PBS (for FRB-FKBP12 pair) for 2 hour at RT. Plates were washed three times with PBS. 50 µl of supernatant from the pACYC-GST clones and 50µl supernatant from the pIT2 clone was mixed with 50 µl of 2% Tween/PBS or 2%BSA/PBS and incubated for 1.5 hours at RT. For detection of the FRB-FKBP 12 pair the assay was performed in the presence of either 0.1 ng/ml rapamycin or no rapamycin. Plates were washed five times with PBST and incubated with Protein L HRP conjugate (1/4000) for I hour at RT. Plates were washed five times with PBST and reactions were developed with 3,3',5,5'-tetramethylbenzidine and stopped with sulphuric acid.

### Results

The direct screening strategy clearly demonstrates the specific binding of M to anti-M. and T to anti-T (Figure 3) and the specific binding of FRB-FKBP12 in the presence of rapamycin (Figure 4).

### References

Andersen, P. S., Stryhn, A., Hansen, B. E., Fugger, L., Engberg, J. & Buus, S. (1996). A recombinant antibody with the antigen-specific, major histocompatibility complex-restricted specificity of T cells. Proc.Natl.Acad.Sci. U.S.A. 93, 1820-1824.
Bussow, K., Cahill, D., Nietfeld, W., Bancroft, D., Scherzinger, E., Lehrach, H. & Walter, G. (1998). A method for global protein expression and antibody screening on high- density filters of an arrayed cDNA library. Nucleic Acids Res 26, 5007-8.
Cai. X. & Garen, A, (1996). A melanoma-specific VH antibody cloned from a fusion phage library of a vaccinated melanoma patient. Proc.Natl.Acad.Sci. USA 93, 6280-6285.
Cho, R. J.. Campbell, M. J., Winzeler, E. A.. Steinmetz, L., Conway, A., Wodicka. L., Wolfsberg, T. G., Gabrielian, A. E., Landsman, D., Lockhart, D. J. & Davis, R. W. (1998). A genome-wide transcriptional analysis of the mitotic cell cycle. Mol Cell 2, 65-73.
Chu, S.. DeRisi. J., Eisen. M., Mulholland. J., Botstein, D., Brown, P. O. & Herskowitz, I. (1998). The transcriptional program of sporulation in budding yeast. Science 282, 699-705.
Clackson, T., Hoogenboom, H. R., Griffiths, A. D. & Winter, G. (1991). Making antibody fragments using phage display libraries. Nature 352, 624-628.
De Kruif, J., Terstappen, L., Boel, E. & Logtenberg, T. (1995). Rapid selection of cell subpopulation-specific human monoclonal antibodies from a synthetic phage antibody library. Proc.Natl.Acad.Sci.USA 92, 3938-3942.
De Wildt, R. M. T., Finnern, R., Ouwehand, W. H., Griffiths, A. D., Van Venrooij, W. J. & Hoet, R. M. A. (1996). Characterization of human variable domain antibody fragments against the U1 RNA-associated A protein, selected from a synthetic and a patient-derived combinatorial V gene library. Eur.J.Immunol. 26, 629-639.
De Wildt, R. M. T., Ruytenbeek, R., Van Venrooij, W. J. & Hoet, R. M. A. (1997). Heavy chain CDR3 optimisation of a germline encoded recombinant antibody fragment predisposed to bind the U1A protein. Protein Eng. 10, 835-841.
DeRisi, J., Penland, L., Brown, P. O., Bittner, M. L., Meltzer, P. S., Ray, M.. Chen, Y., Su, Y. A. & Trent, J. M. (1996). Use of a cDNA microarray to analyse gene expression patterns in human cancer [see comments]. Nat Genet 14, 457-60.
DeRisi, J. L., Iyer, V. R. & Brown, P. O. (1997). Exploring the metabolic and genetic control of gene expression on a genomic scale. Science 278, 680-6.
Griffiths, A. D., Malmqvist, M., Marks, J. D., Bye, J. M., Embleton, M. J., McCafferty, J., Baier, M., Holliger, K. P., Gorick, B. D., Hughes Jones, N. C. & et al . (1993). Human anti-self antibodies with high specificity from phage display libraries. EMBO J. 12, 725-734.
Griffiths, A. D., Williams. S. C., Hartley. O., Tomlinson, I. M., Waterhouse, P., Crosby, W. L., Kontermann, R. E., Jones, P. T., Low, N. M., Allison, T. J.. Prospero. T. D., Hoogenboom, H. R., Nissim, A., Cox, J. P. L., Harrison, J. L., Zaccolo, M., Gherardi, E. & Winter, G. (1994). Isolation of high affinity human antibodies directly from large synthetic repertoires. EMBO J. 13. 3245-3260.
Hoogenboom. H. R., Lutgerink, J. T., Pelsers, M. M., Rousch. M. J., Coote, J., Van Neer, N., De Bruine, A., Van Nieuwenhoven. F. A., Glatz, J. F. & Arends, J. W. (1999). Selection-dominant and nonaccessible epitopes on cell-surface receptors revealed by cell-panning with a large phage antibody library. Eur J Biochem 260, 774-84.
Huse. W. D., Sastry, L., Iverson, S. A., Kang, A. S.. Alting-Mees, M., Burton. D. R., Benkovic, S. J. & Lerner, R. A. (1989). Generation of a large combinatorial library of the immunoglobulin repertoire in phage lambda [see comments]. Science 246, 1275-81.
Iyer, V. R., Eisen, M. B., Ross, D. T., Schuler, G.. Moore. T., Lee, J. C. F., Trent, J. M., Staudt, L. M., Hudson, J.. Jr., Boguski, M. S., Lashkari. D.. Shalon, D., Botstein. D. & Brown. P. O. (1999). The transcriptional program in the response of human fibroblasts to serum [see comments]. Science 283, 83-7.
Kristensen, P. & Winter, G. (1998). Proteolytic selection for protein folding using filamentous bacteriophages. Fold Des 3, 321-8.
Lashkari, D. A., DeRisi, J. L., McCusker, J. H., Namath, A. F., Gentile, C., Hwang. S. Y., Brown, P. O. & Davis, R. W. (1997). Yeast microarrays for genome wide parallel genetic and gene expression analysis. Proc Natl Acad Sci USA 94, 13057-62.
Lueking, A.. Horn. M.. Eickhoff, H., Bussow. K., Lehrach, H. & Walter. G. (1999). Protein microarrays for gene expression and antibody screening [In Process Citation]. Anal Biochem 270, 103-11.
Marks, J. D., Griffiths, A. D., Malmqvist, M., Clackson, T. P., Bye, J. M. & Winter, G. (1992). By-passing immunization: building high affinity human antibodies by chain shuffling. Biotechnology N.Y 10, 779-783.
Marks. J. D.. Hoogenboom. H. R.. Bonnert. T. P., McCafferty. J., Griffiths, A. D. & Winter, G. (1991). By-passing immunization. Human antibodies from V-gene libraries displayed on phage. J Mol. Biol. 222, 581-597.
Mullinax, R. L., Gross, E. A.. Amberg, J. R., Hay, B. N.. Hogrefe, H. H.. Kubitz, M. M., Greener, A., Alting-Mees, M., Ardourel, D., Short. J. M. & et al. (1990). Identification of human antibody fragment clones specific for tetanus toxoid in a bacteriophage lambda immunoexpression library. Proc Natl Acad Sci USA 87, 8095-9.
Osbourn, J. K.. Derbyshire, E. J., Vaughan, T. J., Field, A. W. & Johnson. K. S. (1998). Pathfinder selection: in situ isolation of novel antibodies. Immunotechnology 3, 293-302.
Schier, R., Bye, J., Apell, G., Mccall, A., Adams, G. P., Malmqvist, M., Weiner, L. M. & Marks, J. D. (1996). Isolation of high-affinity monomeric human Anti-c-erbB-2 single chain Fv using affinity-driven selection. J.Mol. Biol. 255, 28-43.
Shalon, D., Smith, S. J. & Brown, P. O. (1996). A DNA microarray system for analyzing complex DNA samples using two- color fluorescent probe hybridisation. Genome Res 6. 639-45.
Sheets. M. D., Amersdorfer, P., Finnern. R., Sargent, P., Lindqvist, E., Schier, R., Hemingsen, G., Wong, C., Gerhart, J. C. & Marks, J. D. (1998). Efficient construction of a large nonimmune phage antibody library: the production of high-affinity human single-chain antibodies to protein antigens. Proc Natl Acad Sci USA 95, 6157-62.
Skerra, A., Dreher. M. L. & Winter, G. (1991). Filter screening of antibody Fab fragments secreted from individual bacterial colonies: specific detection of antigen binding with a two-membrane system. Anal.Biochem. 196, 151-155.
Trent. J. M., Bittner, M., Zhang, J., Wiltshire, R., Ray, M., Su, Y., Gracia, E., Meltzer, P., De Risi, J., Penland, L. & Brown, P. (1997). Use of microgenomic technology for analysis of alterations in DNA copy number and gene expression in malignant melanoma. Clin Exp Immunol 107 Suppl 1, 33-40.
Vaughan, T. J., Williams, A. J., Pritchard, K., Osbourn, J. K., Pope, A. R., Earnshaw, J. C., McCafferty, J., Hodits, R. A., Wilton, J. & Johnson, K. S. (1996). Human antibodies with sub-nanomolar affinities isolated from a large non-immunized phage display library. Nat. Biotechnol. 14, 309-314.
Verheijen, R., Van den Hoogen, F., Beijer, R., Richter, A., Penner, E., Habets, W. J. & van Venrooij, W. J. (1990). A recombinant topoisomerase I used for autoantibody detection in sera from patients with systemic sclerosis. Clin Exp Immunol 80, 38-43.
Watkins. J. D., Beuerlein, G.. Wu, H., McFadden, P. R., Pancook, J. D. & Huse. W. D. (1998). Discovery of human antibodies to cell surface antigens by capture lift screening of phage-expressed antibody libraries. Anal Biochem 256, 169-77.
Welford, S. M., Gregg, J., Chen, E., Garrison, D., Sorensen, P. H., Denny, C. T. & Nelson, S. F. (1998). Detection of differentially expressed genes in primary tumour tissues using representational differences analysis coupled to microarray hybridisation. Nucleic Acids Res 26, 3059-65.
Wodicka, L., Dong, H., Mittmann, M., Ho, M. H. & Lockhart, D. J. (1997). Genome-wide expression monitoring in Saccharomyces cerevisiae. Nat Biotechnol 15, 1359-67.

Although the invention has been described in connection with specific preferred embodiments, it should be understood that the invention as claimed should not be unduly limited to such specific embodiments.

## Claims

1. A method for screening a repertoire of polypeptides to identify one or more members thereof which interact with one or more target molecules, comprising:
a) immobilising the target molecule(s) on a support
b) arraying a plurality of nucleic acid molecules encoding the repertoire of polypeptides;
c) juxtaposing the target molecule(s) and the arrayed nucleic acid molecules;
d) expressing the arrayed nucleic acid molecules to produce the polypeptides such that said polypeptides come into contact with the target molecule(s) on the support and a subset of the polypeptides interact with the target molecule(s): and
e) detecting the interaction of the polypeptides with the target molecule(s) on the support,
wherein the nucleic acid molecules encoding the repertoire of polypeptides and the immobilised target molecules are arrayed and the support/s are juxtaposed prior to expression of the arrayed nucleic acid molecules.

2. A method according to claim 1 wherein the nucleic acid molecules are in the form of expression vectors which encode the members of the repertoire of polypeptides operatively linked to control sequences sufficient to direct their transcription.

3. A method according to claim 2, wherein the expression vector is a bacteriophage.

4. A method according to claim 2, wherein the expression vector is a plasmid.

5. A method according to claim 2, wherein the expression vector is a linear nucleic acid molecule.

6. A method according to any preceding claim, wherein the interaction is selected from the group consisting of a binding interaction; a signalling event, a catalytic reaction; an enzymatic reaction; a phosphorylation event; a glycosylation event; proteolytic cleavage; and a chemical reaction.

7. A method according to any preceding claim, wherein the repertoire of polypeptides is a repertoire of immunoglobulin molecules.

8. A method according to any preceding claim, wherein the nucleic acids are contained and expressed within cells.

9. A method according to claim 8, wherein the cells are selected from the group consisting of bacterial cells, lower eukaryotic cells and higher eukaryotic cells.

10. A method according to any one of claims 1 to 7, wherein the nucleic acid molecules are immobilised in the form of naked or complexed nucleic acid.

11. A method according to any preceding claim, wherein the nucleic acid molecules encoding the repertoire of polypeptides is arrayed onto a different support to the support containing the target molecule(s),

12. A method according to claim 11, wherein the support containing the array of nucleic acid molecules encoding the repertoire of polypeptides is permeable to the polypeptides of the repertoire.

13. A method according to any one of claims 1 to 10, wherein the nucleic acid molecules encoding the repertoire of polypeptides are arrayed onto the same support that contains the immobilised target molecule(s),

14. A method according to any preceding claim, wherein the supports are filter membranes.

15. A method according to any preceding claim, wherein the target molecule(s) are selected from the group consisting of: a purified protein; a recombinant protein; a polypeptide; an enzyme; a substrate for an enzyme; an amino acid; whole cells: a cell extract; a small organic molecule; and a metal ion.

16. A method according to any preceding claim, wherein the repertoire of polypeptides is screened separately against two or more target molecules.

17. A method according to any preceding claim, wherein two or more duplicate arrays of nucleic acid molecules are produced and each is juxtaposed with a different target molecule(s),

18. A method according to claim 1 for screening a repertoire of antibody polypeptides to identify one or more members thereof which bind to one or more target molecules, comprising:
a) immobilising the target molecule(s) on a first support;
b) transforming a plurality of cells with nucleic acid molecules encoding the repertoire of antibody polypeptides and arraying the bacterial cells on a second support;
c) juxtaposing the first and second supports;
d) expressing the nucleic acid molecules to produce the antibody polypeptides such that said polypeptides are secreted from the cells on the second support and come into contact with the target molecule(s) on the first support and a subset of the polypeptides binds to the target molecules; and
e) detecting the binding of the antibody polypeptides with the target molecules on the first support,

19. A method for screening two repertoires of polypeptides against one another to isolated specific binding pairs, which method comprises:
a) immoblising a first generic ligand on a support that is able to bind to all, or substantially all, members of the first repertoire;
b) arraying a plurality of nucleic acid molecules, whereby each point of the array contains a member of the first repertoire and a member of the second repertoire, that encode polypeptides from the first and second repertoires respectively,
c) juxtaposing the first generic ligand and the arrayed nucleic acid molecules;
d) expressing the arrayed nucleic acid molecules to produce an array of polypeptides such that the polypeptide of the first repertoire binds to the first generic ligand and a subset of the polypeptides of the second repertoire binds to the polypeptides of the first repertoire; and
e) detecting the interaction of the second polypeptide with the first polypeptide on the support using a second generic ligand that is able to bind to substantially all members of the second repertoire,
wherein the nucleic acid molecules encoding the repertoire of polypeptides and the immobilised target molecules are arrayed and the support/s are juxtaposed prior to expression of the arrayed nucleic acid molecules.

20. A method for screening a repertoire of enzymes to identify one or more members thereof which convert a substrate molecule to a product molecule, which method comprises:
a) immobilising a ligand on a first support, that is able to bind only to product molecules;
b) arraying a plurality of nucleic acid molecules onto a second support, such that each point of the array contains a nucleic acid member of the enzyme repertoire and the substrate molecule;
c) juxtaposing the first and second supports;
d) expressing the arrayed nucleic acid molecules to produce an array of enzyme polypeptides such that the enzyme polypeptide convert the substrate molecule to a product molecule such that the product molecule is able to bind the specific ligand immobilised on the first support; and
e) detecting the presence of the product molecule on the first support.

21. A method according to claim 20, whereby the substrate molecules are also encoded by a repertoire of polypeptides and each point of the array contains different combinations of members of the enzyme and substrate repertoires.

22. A method for screening a repertoire of substrates to identify one or more members thereof which are converted by an enzyme molecule to form product polypeptides, which method comprises:
a) immobilising a ligand on a first support, that is able to bind only to the product polypeptides;
b) arraying a plurality of nucleic acid molecules onto a second support, such that each point of the array contains a nucleic acid member of the substrate repertoire and the enzyme molecule;
c) juxtaposing the first and second supports;
d) expressing the arrayed nucleic acid molecules to produce an array of substrate polypeptides such that the enzyme molecule converts the substrate polypeptides to product polypeptides such that the product polypeptides is able to bind the specific ligand immobilised on the first support; and
e) detecting the presence of the product polypeptide on the first support.

23. A method according to claim 22, whereby the enzyme molecules are also encoded by a repertoire ofpolypeptides and each point of the array contains different combinations of members of the substrate and enzyme repertoires.

24. A method according to any of claims 20 to 23, whereby the ligand immobilised on the first support binds to both substrate and product molecules and then a product and/or substrate specific detection is used to discriminate between the presence of substrate or product.

25. A method according to claim 1 which comprises the further steps:
f) isolating a nucleic acid encoding a polypeptide that interacts with one or more targets;
g) expressing the polypeptide; and
h) isolating the expressed polypeptide,

## Patentansprüche

1. Verfahren zum Screening einer Gruppe von Polypeptiden, um ein oder mehrere Mitgliede daraus, die mit einem oder mehreren Zielmolekülen wechselwirken, zu identifizieren, bei dem:
a) Das (die) Zielmolekül(e) auf einem Träger immobilisiert werden,
b) eine Vielzahl von Nukleinsäuremolekülen, die die Gruppe der Polypeptide kodieren, in einem Array angeordnet werden,
c) das (die) Zielmolekül(e) und die als Array angeordneten Nukleinsäuremoleküle zueinandergebracht werden,
d) die in einem Array angeordneten Nukleinsäuremoleküle expremiert werden, um die Polypeptide zu erzeugen, so dass die Polypeptide Kontakt mit dem (den) Zielmolekül (en) auf dem Träger kommen und eine Teilmenge der Polypeptide mit dem (den) Zielmolekül(en) wechselwirkt, und
e) die Wechselwirkung der Polypeptide mit dem (den) Zielmolekül(en) auf dem Träger detektiert wird,
wobei sowohl die die Gruppe der Polypeptide kodierenden Nukleinsäuremoleküle als auch die immobilisierten Zielmoleküle in einem Array angeordnet werden und der (die) Träger vor der Expression der in einem Array angeordneten Nukleinsäuremoleküle zusammengebracht werden.

2. Verfahren nach Anspruch 1, wobei die Nukleinsäuremoleküle in Form von Expressionsvektoren vorliegen, die Mitglieder der Gruppe der Polypeptide kodieren, wobei sie funktionsmäßig ausreichend mit Kontrollsequenzen verbunden sind, um ihre Transkreption zu steuern.

3. Verfahren nach Anspruch 2, wobei der Expressionsvektor ein Bakteriophage ist.

4. Verfahren nach Anspruch 2, wobei der Expressionsvektor ein Plasmid ist.

5. Verfahren nach Anspruch 2, wobei der Expressionsvektor ein lineares Nukleinsäuremolekül ist.

6. Verfahren nach einem der vorhergehenden Ansprüche, wobei die Wechselwirkung aus der Gruppe ausgewählt ist, die besteht aus: Eine Bindungswechselwirkung, ein Signalereignis, eine katalytische Reaktion, eine enzymatische Reaktion, ein Phosphorilierungsereignis, ein Glycosilationsereignis, proteolytische Spaltung und eine chemische Reaktion.

7. Verfahren nach einem der vorhergehenden Ansprüche, wobei die Gruppe der Polypeptide eine Gruppe von Immunoglobolinmolekülen ist.

8. Verfahren nach einem der vorhergehenden Ansprüche, wobei die Nukleinsäure in Zellen enthalten sind und darin expremiert werden.

9. Verfahren nach Anspruch 8, wobei die Zellen aus der Gruppe ausgewählt sind, die aus bakteriellen Zellen, niederen eukaryotischen Zellen und höheren eukaryotischen Zellen besteht.

10. Verfahren nach einem der Ansprüche 1 bis 7, wobei die Nukleinsäuremoleküle in Form von nackten oder komplexierten Nukleinsäuren immobilisiert werden.

11. Verfahren nach einem der vorhergehenden Ansprüche, wobei die die Gruppe der Polypeptide kodierenden Nukleinsäuremoleküle in einem Array auf einem anderen Träger als demjenigen Träger angeordnet werden, der das (die) Zielmolekül(e) enthält.

12. Verfahren nach Anspruch 11, wobei der das Array der die Gruppe von Polypeptiden kodierenden Nukleinsäuremoleküle enthaltende Träger für die Polypeptide der Gruppe permeabel ist.

13. Verfahren nach einem der Anspruche 1 bis 10, wobei die die Gruppe von Polypeptiden kodierenden Nukleinsäuremoleküle auf demselben Träger als Array angeordnet werden, der das (die) immobilisierte(n) Zielmolekül(e) enthält.

14. Verfahren nach einem der vorhergehenden Ansprüche, wobei die Träger Filtermembranen sind.

15. Verfahren nach einem der vorgehenden Ansprüche, wobei das (die) Zielmolekül(e) aus der Gruppe ausgewählt ist (sind), die besteht aus: Einem gereinigten Protein, einem rekombinanten Protein, einem Polypeptid, einem Enzym, einem Substrat für ein Enzym, einer Aminosäure, ganzen Zellen, einem Zellextrakt, einem kleinen organischen Molekül und einem Metallion.

16. Verfahren nach einem der vorhergehenden Ansprüche, wobei die Gruppe der Polypeptide separat gegen zwei oder mehr Zielmoleküle gescreent werden.

17. Verfahren nach einem der vorhergehenden Ansprüche, wobei zwei oder mehrere duplizierte Arrays von Nukleinsäuremolekülen erzeugt werden und mit einem verschiedenen Zielmolekül(en) zusammengebracht werden.

18. Verfahren nach Anspruch 1, zum Screening einer Gruppe von Antikörper-Polypeptiden, um ein oder mehrere Mitglieder davon zu identifizieren, die an ein oder mehrere Zielmoleküle binden, bei dem:
a) Das (die) Zielmolekül(e) auf einem ersten Träger immobilisiert wird (werden),
b) mehrere Zellen mit den die Gruppe von Antikörper-Polypeptiden kodierenden Nukleinsäuremolekülen transformiert werden und die bakteriellen Zellen auf einem zweiten Träger in einem Array angeordnet werden,
c) der erste und der zweite Träger zusammengebracht werden,
d) die Nukleinsäuremoleküle expremiert werden, um die Antikörper-Polypeptide zu erzeugen, so dass die Polypeptide aus den Zellen auf dem zweiten Träger ausgeschieden werden und Kontakt mit dem (den) Zielmolekül(en) auf dem ersten Träger kommen und eine Teilmenge der Polypeptide an die Zielmoleküle bindet, und
e) die Bindung der Antikörper-Polypeptide mit den zielmolekülen auf dem ersten Träger detektiert wird.

19. Verfahren zum Screening von zwei Gruppen von Polypeptiden gegeneinander auf isolierte spezifische Bindungspaare, wobei bei dem Verfahren:
a) Auf einem Träger ein erster generischer Ligand immobilisiert wird, der dazu in der Lage ist, an alle oder im Wesentlichen alle Mitglieder der ersten Gruppe zu binden,
b) eine Vielzahl von Nukleinsäuremolekülen in einem Array angeordnet wird, wodurch jeder Punkt des Arrays ein Mitglied der ersten Gruppe und ein Mitglied der zweiten Gruppe enthält, die Polypeptide der ersten respektive der zweiten Gruppe kodieren,
c) der erste generische Ligand und die in Arrays angeordneten Nukleinsäuremoleküle zusammengebracht werden,
d) die als Arrays angeordneten Nukleinsäuremoleküle expremiert werden, um ein Array von Polypeptiden zu erzeugen, so dass die Polypeptide der ersten Gruppe an dem ersten generischen Liganden binden und eine Teilmenge der Polypeptide der zweiten Gruppe an den Polypeptiden der ersten Gruppe bindet, und
e) die Wechselwirkung des Polypeptids mit dem ersten Polypeptid auf dem Träger unter Verwendung eines zweiten generischen Liganden detektiert wird, der dazu in der Lage ist, an im Wesentlichen alle Mitglieder der zweiten Gruppe zu binden,
wobei die die Gruppe der Polypeptide kodierenden Nukleinsäuremoleküle und die immobisierten Zielmoleküle als Arrays angeordnet werden und der (die) Träger vor der Expression der als Arrays angeordneten Nukleinsäuremoleküle zusammengebracht werden.

20. Verfahren zum Screening einer Gruppe von Enzymen, um ein oder mehrere Mitglieder davon zu identifizieren, die ein Substratmolekül in ein Produktmolekül umwandeln, wobei bei dem Verfahren:
a) Auf einem ersten Träger ein Ligand immobilisiert wird, der dazu in der Lage ist, nur an Produktmoleküle zu binden,
b) eine Vielzahl von Nukleinsäuremolekülen auf einem zweiten Träger als Array angeordnet wird, so dass jeder Punkt des Arrays ein Nukleinsäuremitglied der Enzymgruppe und das Substratmolekül enthält,
c) die ersten und zweiten Träger zusammengebracht werden,
d) die als Array angeordneten Nukleinsäuremoleküle expremiert werden, um ein Array aus Enzympolypeptiden zu erzeugen, so dass die Enzympolypeptide das Substratmolekül in ein Produktmolekül umwandeln, so dass das Produktmolekül dazu in der Lage ist, an den spezifischen Liganden zu binden, der auf dem ersten Träger immobilisiert ist, und
e) das Vorhandensein des Produktmoleküls auf dem ersten Träger detektiert wird.

21. Verfahren nach Anspruch 20, wobei die Substratmoleküle auch durch eine Gruppe von Polypeptiden kodiert werden und jeder Punkt des Arrays verschiedene Kombinationen von Mitgliedern der Enzymgruppe und der Substratgruppe enthalten.

22. Verfahren zum Screening einer Gruppe von Substraten, um ein oder mehrere Mitglieder davon zu identifizieren, die durch ein Enzymmolekül umgewandelt werden, um Produktpolypeptide zu bilden, wobei bei dem Verfahren:
a) Auf einem ersten Träger ein Ligand immobilisiert wird, der dazu in der Lage ist, nur an die Produktpolypeptide zu binden,
b) eine Vielzahl von Nukleinsäuremolekülen auf einem zweiten Träger als Array angeordnet wird, so dass jeder Punkt des Array ein Nukleinsäuremitglied der Substratgruppe und das Enzymmolekül enthält,
c) die ersten und zweit Träger zusammengebracht werden,
d) die als Array angeordneten Nukleinsäuremoleküle expremiert werden, um ein Array von Substratpolypeptiden zu erzeugen, so dass das Enzymmolekül die Substratpolypeptide umwandelt, um Produktpolypeptide zu erzeugen, so dass die Produktpolypeptide dazu in der Lage sind, an den auf dem ersten Träger immobilisierten spezifischen Liganden zu binden, und
e) das Vorhandensein der Produktpolypeptide auf dem ersten Träger detektiert wird.

23. Verfahren nach Anspruch 22, wobei die Enzymmoleküle auch durch eine Gruppe von Polypeptiden kodiert werden und jeder Punkt des Arrays verschiedene Kombinationen von Mitgliedern der Substratgruppe und der Enzymgruppe enthält.

24. Verfahren nach einem der 20 bis 23, wobei der auf dem ersten Träger immobilisierte Ligand sowohl an Substrat- als auch an bindet und dann eine Produkt- und/oder substratspezifische Detektion verwendet wird, um zwischen dem Substrat oder dem Produkt zu unterscheiden.

25. Verfahren nach Anspruch 1, bei dem weiter:
f) Eine ein Polypeptid, as einem oder mehreren Zielen wechselwirkt, kodierende Nukleinsäure isoliert wird,
g) das Polypeptid expremiert wird, und
h) das expremierte Polypeptid isoliert wird.

## Revendications

1. Procédé de criblage d'un répertoire de polypeptides pour identifier un ou plusieurs membres de celui-ci qui interagisse avec une ou plusieurs molécules cibles, comprenant :
a) l'immobilisation de la (des) molécule(s) cible(s) sur un support ;
b) le dépôt en réseau d'une pluralité de molécules d'acide nucléique codant pour le répertoire de polypeptides ;
c) la juxtaposition de la (des) molécule(s) cible(s) et des molécules d'acide nucléique déposées en réseau ;
d) l'expression des molécules d'acide nucléique déposées en réseau pour produire les polypeptides de telle manière que lesdits polypeptides entrent en contact avec la (les) molécule(s) cible(s) sur le support et qu'un sous-ensemble des polypeptides interagit avec la (les) molécule(s) cible(s) ; et
e) la détection de l'interaction des polypeptides avec la (les) molécule(s) cible(s) sur le support,
dans lequel les molécules d'acide nucléique codant pour le répertoire de polypeptides et les molécules cibles immobilisées sont déposées en réseau et le(s) support(s) sont juxtaposés avant l'expression des molécules d'acide nucléique déposées en réseau.

2. Procédé selon la revendication 1, dans lequel les molécules d'acide nucléique sont sous la forme de vecteurs d'expression qui codent pour les membres du répertoire de polypeptides liés de manière fonctionnelle à des séquences contrôles suffisantes pour diriger leur transcription.

3. Procédé selon la revendication 2, dans lequel le vecteur d'expression est un bactériophage.

4. Procédé selon la revendication 2, dans lequel le vecteur d'expression est un plasmide.

5. Procédé selon la revendication 2, dans lequel le vecteur d'expression est une molécule d'acide nucléique linéaire.

6. Procédé selon l'une quelconque des revendications précédentes dans lequel l'interaction est choisie dans le groupe constitué : d'une interaction de liaison ; d'un événement de signalisation, d'une réaction catalytique ; d'une réaction enzymatique ; d'un événement de phosphorylation ; d'un événement de glycosylation ; d'un clivage protéolytique ; et d'une réaction chimique.

7. procédé selon l'une quelconque des revendications précédentes, dans lequel le répertoire de polypeptides est un répertoire de molécules d'immunoglobulines.

8. Procédé selon l'une quelconque des revendications précédentes, dans lequel les acides nucléiques sont contenus et exprimés au sein de cellules.

9. Procédé selon la revendication 8, dans lequel les cellules sont choisies dans le groupe constitué de cellules bactériennes, de cellules d'eucaryotes inférieurs et de cellules d'eucaryotes supérieures.

10. Procédé selon l'une quelconque des revendications 1 à 7, dans lequel les molécules d'acide nucléique sont immobilisées sous la forme d'acide nucléique nu ou complexé.

11. Procédé selon l'une quelconque des revendications précédentes, dans lequel les molécules d'acide nucléique codant pour le répertoire de polypeptides sont déposées en réseau sur un support différent du support contenant la (les) molécule (s) cible(s).

12. Procédé selon la revendication 11, dans lequel le support contenant le réseau des molécules d'acide nucléique codant pour le répertoire de polypeptides est perméable aux polypeptides du répertoire.

13. Procédé selon l'une quelconque des revendications 1 à 10, dans lequel les molécules d'acide nucléique codant pour le répertoire de polypeptides sont déposée en réseau sur le même support qui contient la (les) molécule(s) cible(s) immobilisée(s).

14. Procédé selon l'une quelconque des revendications précédentes, dans lequel les supports sont des membranes filtrantes.

15. Procédé selon l'une quelconque des revendications précédentes, dans lequel la (les) molécule(s) cible(s) est (sont) choisie(s) dans le groupe constitué : d'une protéine purifiée ; d'une protéine recombinante ; d'un polypeptide ; d'une enzyme ; d'un substrat pour une enzyme ; d'un acide aminé ; de cellules entières ; d'un extrait cellulaire ; d'une petite molécule organique ; et d'un ion métallique.

16. Procédé selon l'une quelconque des revendications précédentes dans lequel le répertoire de polypeptides est criblé séparément contre deux molécules cibles ou plus.

17. Procédé selon l'une quelconque des revendications précédentes, dans lequel deux réseaux en double ou plus de molécules d'acide nucléique sont produits et chacun est juxtaposé avec une (des) molécule(s) cible(s) différente(s).

18. Procédé selon la revendication 1 pour le criblage d'un répertoire de polypeptides anticorps pour identifier un ou plusieurs membres de celui-ci qui se lient à une ou plusieurs molécules cibles, comprenant :
a) l'immobilisation de la (des) molécule(s) cible(s) sur un premier support ;
b) la transformation d'une pluralité de cellules avec des molécules d'acide nucléique codant pour le répertoire de polypeptide anticorps et le dépôt en réseau des cellules bactériennes sur un second support ;
c) la juxtaposition des premier et second supports ;
d) l'expression des molécules d'acide nucléique pour produire les polypeptides anticorps de telle manière que lesdits polypeptides sont sécrétés à partir des cellules sur le second support et entrent en contact avec la (les) molécule(s) cible(s) sur le premier support et qu'un sous-ensemble des polypeptides se lie aux molécules cibles ; et
e) la détection de la liaison des polypeptides anticorps avec les molécules cibles sur le premier support.

19. Procédé de criblage de deux répertoires de polypeptides l'un contre l'autre pour isoler des paires de liaisons spécifiques, lequel procédé comprend :
a) l'immobilisation d'un premier ligand générique sur un support qui est capable de se lier à la totalité, ou pratiquement la totalité, des membres du premier répertoire ;
b) le dépôt en réseau d'une pluralité de molécules d'acide nucléique, moyennant quoi chaque point du réseau contient un membre du premier répertoire et un membre du second répertoire, qui codent pour des polypeptides provenant, respectivement, des premier et second répertoires ;
c) la juxtaposition du premier ligand générique et des molécules d'acide nucléique déposées en réseau ;
d) l'expression des molécules d'acide nucléique déposées en réseau pour produire un réseau de polypeptides de telle manière que le polypeptide du premier répertoire se lie au premier ligand générique et qu'un sous-ensemble des polypeptides du second répertoire se lie aux polypeptides du premier répertoire ; et
e) la détection de l'interaction du second polypeptide avec le premier polypeptide sur le support en utilisant un second ligand générique qui est capable de se lier à pratiquement tous les membres du second répertoire,
dans lequel les molécules d'acide nucléique codant pour le répertoire de polypeptides et les molécules cibles immobilisées sont déposées en réseau et le (s) support(s) est (sont) juxtaposé(s) avant l'expression des molécules d'acide nucléique déposées en réseau.

20. Procédé de criblage d'un répertoire d'enzymes pour identifier un ou plusieurs membres de celui-ci qui convertissent une molécule de substrat en une molécule de produit, lequel procédé comprend :
a) l'immobilisation d'un ligand sur un premier support, qui est capable de se lier uniquement aux molécules de produit ;
b) le dépôt en réseau d'une pluralité de molécules d'acide nucléique sur un second support, de telle manière que chaque point du réseau contient un membre d'acide nucléique du répertoire d'enzymes et la molécule de substrat ;
c) la juxtaposition des premier et second supports ;
d) l'expression des molécules d'acide nucléique déposées en réseau pour produire un réseau de polypeptides enzymes de telle manière que le polypeptide enzyme couvert it la molécule de substrat en une molécule de produit de telle manière que la molécule de produit est capable de se lier au ligand spécifique immobilisé sur le premier support ; et
e) la détection de la présence de la molécule de produit sur le premier support.

21. Procédé selon la revendication 20, par lequel les molécules de substrats sont également codées par un répertoire de polypeptides et chaque point du réseau contient des combinaisons différentes des membres des répertoires d'enzymes et de substrats.

22. Procédé de criblage d'un répertoire de substrats pour identifier un ou plusieurs membres de celui-ci qui sont convertis par une molécule d'enzyme pour former des polypeptides produits, lequel procédé comprend :
a) l'immobilisation d'un ligand sur un premier support, qui est capable de se lier uniquement aux polypeptides produits ;
b) le dépôt en réseau des molécules d'acide nucléique sur un second support, de telle manière que chaque point du réseau contient un membre d'acide nucléique du répertoire de substrats et la molécule d'enzyme ;
c) la juxtaposition des premier et second supports ;
d) l'expression des molécules d'acide nucléique déposées en réseau pour produire un réseau de polypeptides substrats de telle manière que la molécule d'enzyme convertit les polypeptides substrats pour produire des polypeptides de telle manière que les polypeptides produits sont capables de se lier au ligand spécifique immobilisé sur le premier support ; et
e) la détection de la présence du polypeptide produit sur le premier support.

23. Procédé selon la revendication 22, par lequel les molécules d'enzyme sont également codées par un répertoire de polypeptides et chaque point du réseau contient des combinaisons différentes des membres des répertoires de substrats et d'enzymes.

24. Procédé selon l'une quelconque des revendications 20 à 23, par lequel le ligand immobilisé sur le premier support se lie aux deux molécules de substrat et de produit et ensuite une détection spécifique du produit et/ou du substrat est utilisée pour faire la discrimination entre la présence de substrat ou de produit.

25. Procédé selon la revendication 1, qui comprend les étapes supplémentaires :
f) d'isolement d'un acide nucléique codant pour un polypeptide qui interagit avec une ou plusieurs cibles ;
g) d'expression du polypeptide ; et
h) d'isolement du polypeptide exprimé.
